# EUROPEAN PATENT APPLICATION

(11) **EP 2 351 763 A1**
(43) Date of publication of application: **03.08.2011**
(21) Application number: 10763575.7
(22) Date of filing: 14.06.2010
(51) Int. Cl.: C07H 7/027, C07H 1/08

(54) **METHOD FOR EXTRACTION OF COMPOUND CONTAINING SIALIC ACID FROM PLANT**

(30) Priority: 06.10.2009 JP 2009232161
(71) Applicant: Incorporated Administrative Agency National Agriculture and Food Research Organization, Ibaraki 305-8517 (JP)
(72) Inventor: Kawase, Shin-ichiro, Hiroshima 7218514 (JP)
(74) Representative: Vossius & Partner
(86) International application number: PCT/JP2010/060010
(87) International publication number: WO 2011/043107

(57) **Abstract**

An obj ect of the present invention is to provide an inexpensive and simple extraction method for a sialic acid-containing compound from a raw material which is easily available and has no risk of contamination with pathogens affecting animals (raw material which has high safety) without requiring the use of a reagent harmful to the human body and environment. The present invention is also aimed at providing an extraction method for a sialic acid-containing compound, the method comprising: crudely extracting a soluble component from a plant body (in particular, seeds of cereal and/or seeds of bean) or a processed product of the plant body with water, an alcohol, or an aqueous alcohol; and separating and recovering the sialic acid-containing compound from the resultant crude extract solution by dialysis, salt precipitation, or column chromatography.

## Description

### Technical Field

The present invention relates to an extraction method for a sialic acid-containing compound from a plant, and more specifically, to an extraction method for a sialic acid-containing compound from seeds of cereal and/or seeds of bean.

### Background Art

Sialic acid is a generic term for acyl derivatives of neuraminic acid, which is an amino sugar, and 20 or more types of derivatives are available. Naturally, sialic acid is present as N-acetylneuraminic acid and N-glycolylneuraminic acid in the largest amounts, and is also present in the brains of animals, cow's milk, chicken egg yolk, and the like as "ganglioside", which is sialic acid-containing sphingoglycolipid and as a "polysialic acid sugar chain", which is a sialic acid-containing sugar chain.
Ganglioside is a generic term for glycolipids each having sialic acid at the non-reducing terminal side, and a plurality of types thereof are available depending on the number and binding sites of sialic acids. Polysialic acid is a sugar chain in which several tens or more of sialic acids are bound to each other. Polysialic acid composes a sugar chain moiety of a neural cell adhesion molecule (NCAM) that is a huge nerve specific glycoprotein that functions in the fields of neural cell migration, neurite elongation, and synapse formation.

A compound including sialic acid in its molecular structure has properties of (1) developing a red purple color or a blue purple color by reacting with a resorcinol hydrochloric acid reagent (see e.g., Non-patent Document 1) and (2) exhibiting affinity to a serotonin affinity column (see e.g., Non-patent Document 2). Conversely, a substance that exhibits those properties is a compound having the sialic acid in its molecular structure.

The sialic acid-containing compound is a substance essential for the differentiation, growth, and maintenance of nerves that compose the brain, and the substance has a high functionality. For example, oral ingestion thereof has been observed to enhance learning ability (see e.g., Non-patent Document 3). In addition, the sialic acid-containing compound has been also known to have immunostimulatory effects and anticancer effects. Therefore in the future, the sialic acid-containing compound is expected to be utilized widely for treatments of brain-related diseases and malignant tumor diseases that will increase along with the coming aging society.

The sialic acid-containing compound has been prepared mainly from bovine brain and cow's milk so far, but it has become difficult to prepare from the bovine brain and the cow's milk owing to the occurrence of bovine spongiform encephalopathy (BSE). In addition, a chicken egg is also a source of the sialic acid-containing compound, but safety in the production of the sialic acid-containing compound from the chicken egg has been doubtful owing to the occurrence of avian influenza. That is, it is hard to say that all the consumers agree with its safety, though it has been described that the chicken egg is not infected with an avian influenza virus.
Note that an attempt by a person to produce the sialic acid-containing compound by chemical synthesis has been made. However, the synthetic technique thereof at present has not attained a practical level in terms of yield and cost, and mass production thereof has not been achieved.
From such circumstances, a new source of the sialic acid-containing compound, which has no risk of contamination with pathogens such as a virus or prion which affects animals and is derived from a natural material, has been desired.
In addition, in conventional methods of extracting the sialic acid-containing compound derived from natural substance, the extraction is performed using a chloroform/methanol mixed solvent, hot methanol, or the like (various organic solvents typified by chloroform are also used in chemical synthesis methods of the sialic acid-containing compound). There have also been moves to reduce the use of the harmful organic solvents in light of environmental problems and effects on human bodies, and thus, the development of an extraction method for the sialic acid-containing compound with due consideration to the problems and effects has been desired.

Reports which suggest that sialic acid is present in plants have been described in the articles of Shar et al. (see Non-Patent Documents 4 and 5) so far.
However, in the articles of Shar et al., there is only the description of indirect evidence that suggests the presence of sialic acid in *Arabidopsis thaliana*, no experiment for directly examining the presence or absence of the nature inherent to the sialic acid-containing compound has been performed, and no specific extraction method is disclosed.

Note that, in Patent Document 1, it has been merely described that glycolipids each including ganglioside can be similarly extracted from raw materials derived from living bodies including the plant. However, only the extraction method from murine brain has been disclosed in detail, and no specific extraction method for the glycolipid including ganglioside from the plant as the raw material has been described. In addition, the method is not preferred because chloroform is used.

### Citation List

### Patent Document

[Patent Document 1] JP 2003-129083 A

### Non-Patent Document

[Non-Patent Document 1] Shin Seikagaku Jikken Koza 4 Lipid III Glycolipid, (1990) p149
[Non-Patent Document 2] Carbohydrate Research, 103 (1982) p213-219
[Non-Patent Document 3] Ganglioside Kenkyuhou I, (1995), p7-25
[Non-Patent Document 4] Nature Biotechnology, 21 (2003) p1470-1471
[Non-Patent Document 5] Nature Biotechnology, 22 (2004) p1351-1352

### Summary of Invention

### Problem to be solved by the Invention

An object of the present invention is to solve the above-mentioned conventional problem and to extract a sialic acid-containing compound easily at low cost from a raw material which is easily available and has no risk of contamination with pathogens affecting animals (raw material which has high safety) without requiring the use of a reagent harmful to the human body and environment.

### Means for solving the Problem

The inventor of the present invention has made extensive studies. As a result, the inventor has found that a large amount of a sialic acid-containing compound is present in a plant body, in particular, seeds of cereal and seeds of bean.
Then, the inventor of the present invention has found that a sialic acid-containing compound which has no risk of contamination with pathogens affecting animals can be easily extracted by crudely extracting a soluble component from the plant body with water, an alcohol, or water containing an aqueous alcohol, and purifying the resultant crude extract solution.
The present invention has been completed based on such findings.

That is, a first aspect of the present invention provides an extraction method for a sialic acid-containing compound, the method comprising: crudely extracting a soluble component from a plant body or a processed product of the plant body with water, an alcohol, or an aqueous alcohol; and separating and recovering the sialic acid-containing compound from the resultant crude extract solution by dialysis, salt precipitation, or column chromatography.
A second aspect of the present invention provides an extraction method for a sialic acid-containing compound according to the first aspect, in which the plant body includes seeds of cereal and/or seeds of bean.
A third aspect of the present invention provides an extraction method for a sialic acid-containing compound according to the first or the second aspect, in which the plant body or the processed product of the plant body has been milled, ground, triturated, or pulverized.
A forth aspect of the present invention provides an extraction method for a sialic acid-containing compound according to any one of the first to third aspects, in which ultrasonication is performed in the step of crudely extracting the soluble component.
A fifth aspect of the present invention provides an extraction method for a sialic acid-containing compound according to any one of the first to fourth aspects, in which the water is tap water, deionized water, distilled water, or water containing a salt, an acid, an alkali, saccharides, or a pH buffer.
A sixth aspect of the present invention provides an extraction method for a sialic acid-containing compound according to any one of the first to fifth aspect, in which the alcohol is methanol, ethanol, isopropanol, ethylene glycol, or glycerol.
A seventh aspect of the present invention provides an extraction method for a sialic acid-containing compound according to any one of the first to sixth aspect, in which the separating and recovering are performed with a serotonin affinity column, a lectin affinity column, a gel filtration column, an ion-exchange column, or a silica gel column.
An eighth aspect of the present invention provides a sialic acid-containing compound, which is obtained by the extraction method according to any one of the first to seventh aspect.

### Effects of the Invention

The present invention can provide the sialic acid-containing compound which has no risk of contamination with pathogens affecting animals and has high safety because the raw material is derived from a plant (in particular, seeds of cereal or seeds of bean) and harmful chemicals and the like (in particular, chloroform) are not used in a production step.
Further, the present invention can provide the sialic acid-containing compound at low cost because the raw material is easily available and processed.
In addition, the present invention allows the effective utilization of cereals or beans which are out of specification or are discharged, wastes which are generated during the processing of cereals (such as rice bran and bran), and beans which are out of specification.

### Brief Description of Drawings

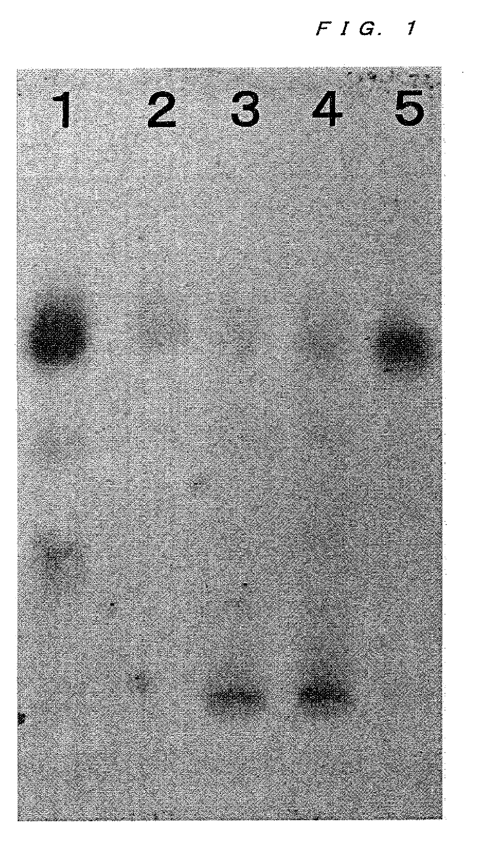

[FIG. 1] FIG. 1 is an image showing the presence of sialic acid-containing compounds detected by a resorcinol hydrochloric acid treatment after crudely extracting from whole soy flour, wheat flour, brown rice whole grain flour, or flour of a brown rice aleurone layer part with water as an extraction solvent, and separating the resultant crude extract solution by thin layer chromatography in Test Examples 1 to 3.

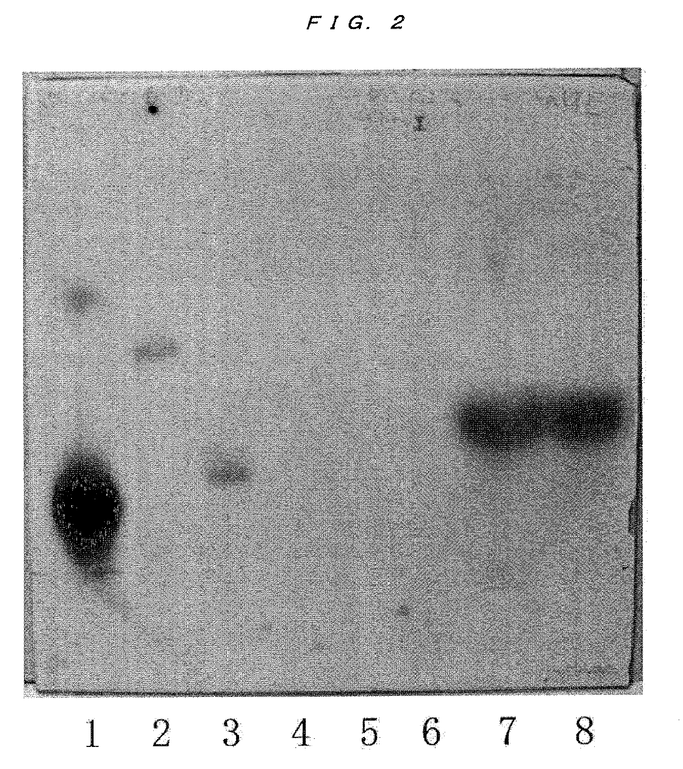

[FIG. 2] FIG. 2 is an image showing the presence of sialic acid-containing compounds detected by the resorcinol hydrochloric acid treatment after crudely extracting from flour of a brown rice aleurone layer part or rice germ with ethylene glycol as the extraction solvent, and separating the resultant crude extract solution by the thin layer chromatography in Test Example 4.

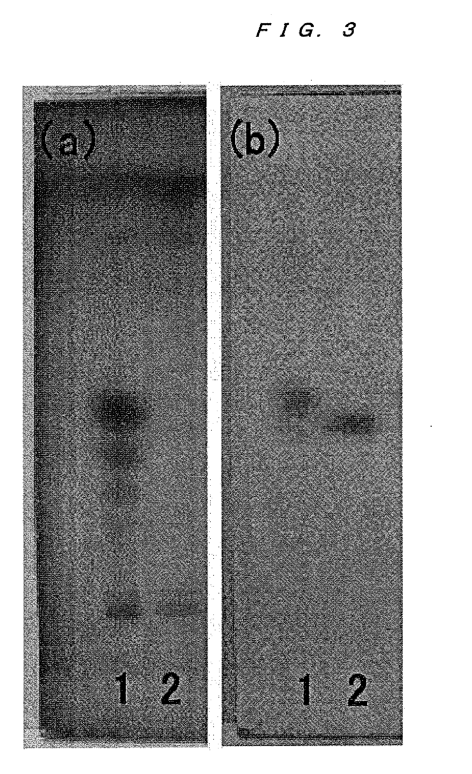

[FIGS. 3] FIG. 3 (a) is an image showing the presence of all organic compounds detected under an iodine vapor atmosphere after crudely extracting from flour of a brown rice aleurone layer part with ethylene glycol or water as the extraction solvent, and separating the resultant crude extract solution by the thin layer chromatography in Test Example 5. FIG. 3 (b) is an image showing the presence of sialic acid-containing compounds detected by the resorcinol hydrochloric acid treatment after separating a crude extract solution extracted from the flour of the brown rice aleurone layer part with ethylene glycol or water as the extraction solvent by the thin layer chromatography in Test Example 5.

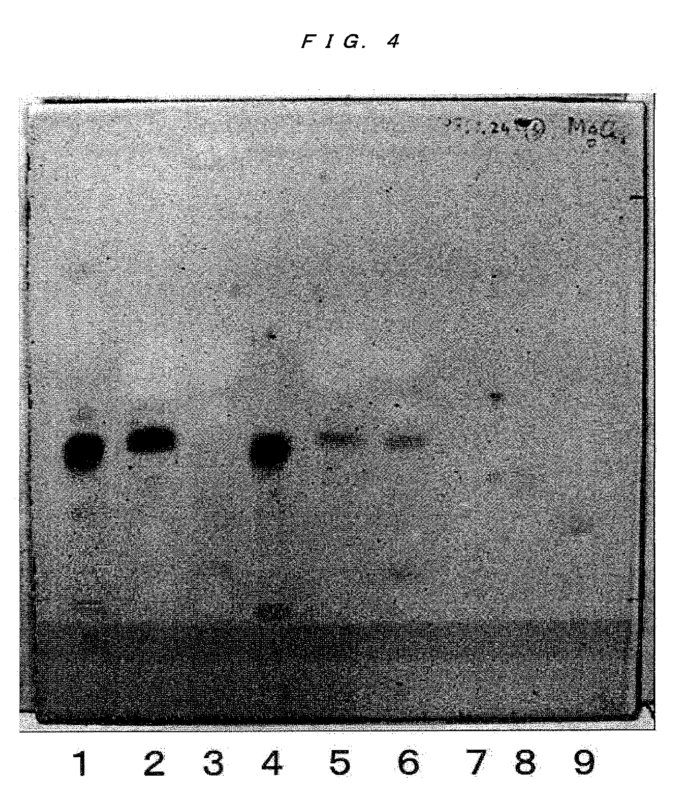

[FIG. 4] FIG. 4 is an image showing the presence of sialic acid-containing compounds detected by the resorcinol hydrochloric acid treatment after crudely extracting from flour of a brown rice aleurone layer part or flour of a polished rice surface layer part with water as the extraction solvent, and separating the resultant crude extract solution by the thin layer chromatography in Test Example 6.

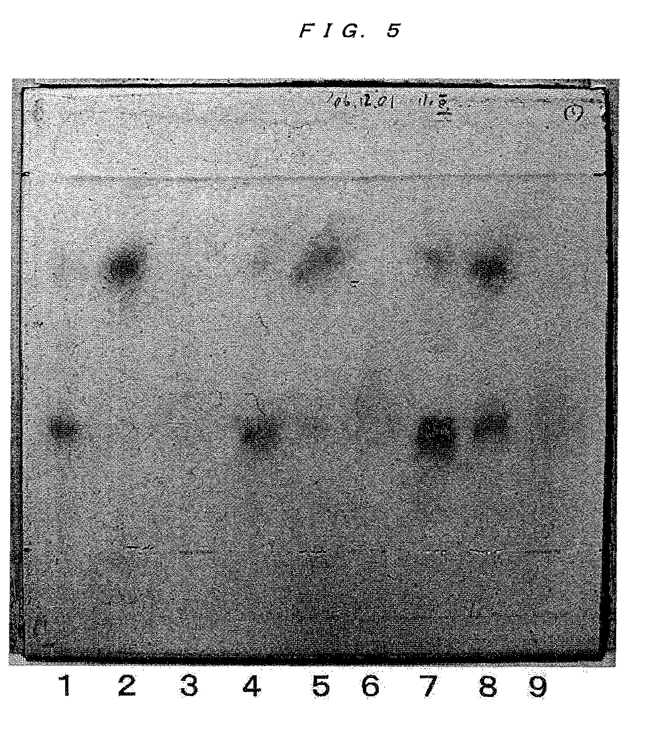

[FIG. 5] FIG. 5 is an image showing the presence of sialic acid-containing compounds detected by the resorcinol hydrochloric acid treatment after crudely extracting from adzuki bean seed flour with water, hydrochloric acid-containing water, or ethylene glycol as the extraction solvent, and separating the resultant crude extract solution by the thin layer chromatography in Test Example 7.

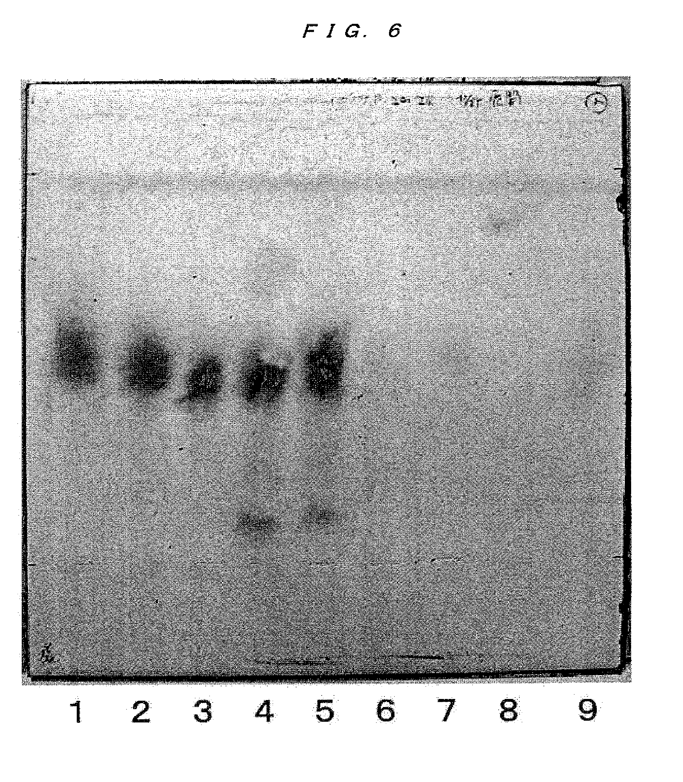

[FIG. 6] FIG. 6 is an image showing the presence of sialic acid-containing compounds detected by the resorcinol hydrochloric acid treatment after crudely extracting from flour of a brown rice outermost layer part, a brown rice aleurone layer part, a polished rice surface layer part, a black soybean seed cotyledon, or corn or maize, and separating the resultant crude extract solution by the thin layer chromatography in Test Examples 8 to 10.

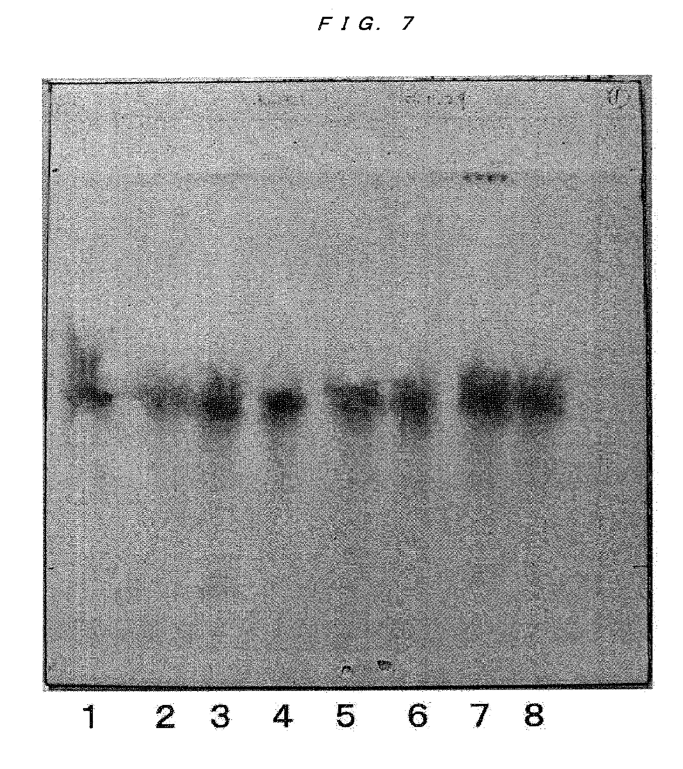

[FIG. 7] FIG. 7 is an image showing the presence of sialic acid-containing compounds detected by the resorcinol hydrochloric acid treatment after crudely extracting from rice germ or flour of a brown rice aleurone layer part with water as the extraction solvent, and separating the resultant crude extract solution by the thin layer chromatography in Test Example 11.

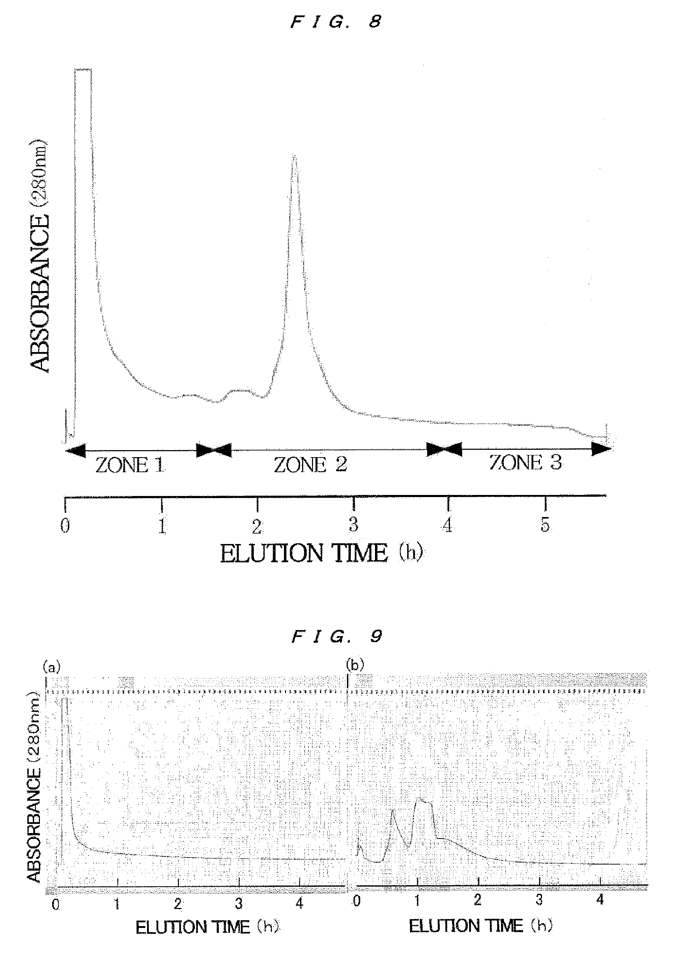

[FIG. 8] FIG. 8 is a chart in the case of extracting from flour of a brown rice aleurone layer part with water, and fractionating the resultant extract solution with a serotonin affinity column in Example 1.
[FIGS. 9] FIGS. 9 are charts each in the case of extracting from rice germ with water, and fractionating the resultant extract solution with the serotonin affinity column in Example 2.

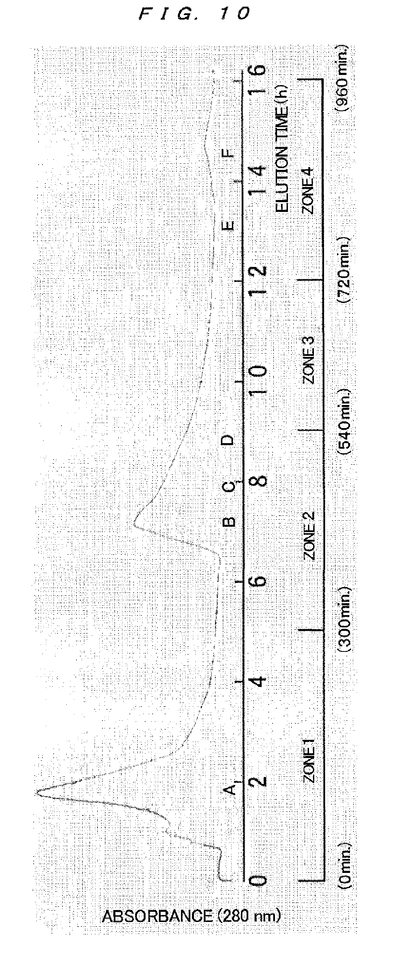

[FIG. 10] FIG. 10 is a chart in the case of extracting from flour of a polished rice surface layer part with water, and fractionating the resultant extract solution with the serotonin affinity column in Example 3.

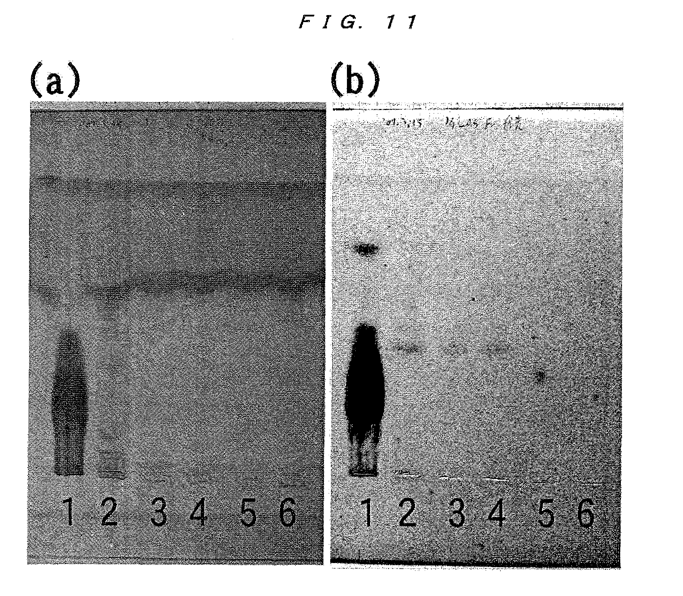

[FIGS. 11] FIG. 11 (a) is an image showing the presence of all organic compounds detected under the iodine vapor atmosphere after separating the fraction fractionated with the serotonin affinity column by the thin layer chromatography in Example 3. FIG. 11 (b) is an image showing the presence of sialic acid-containing compounds detected by the resorcinol hydrochloric acid treatment in Example 3.

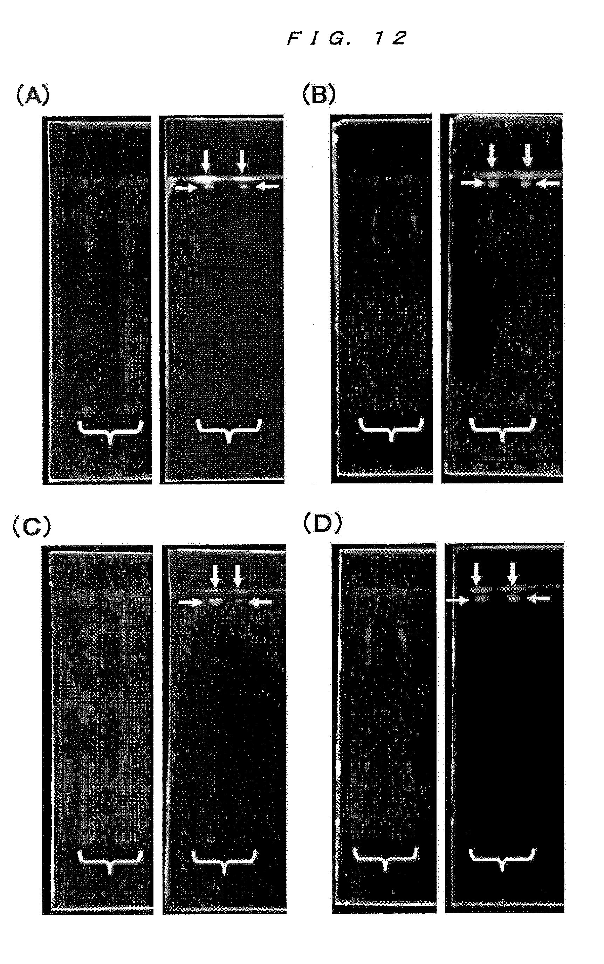

[FIGS. 12] FIGS. 12 are images each showing the presence of sialic acid-containing compounds detected by a lectin binding reaction after crudely extracting from germ with ethylene glycol as the extraction solvent, and separating the resultant crude extract solution by the thin layer chromatography in Test Example 12.

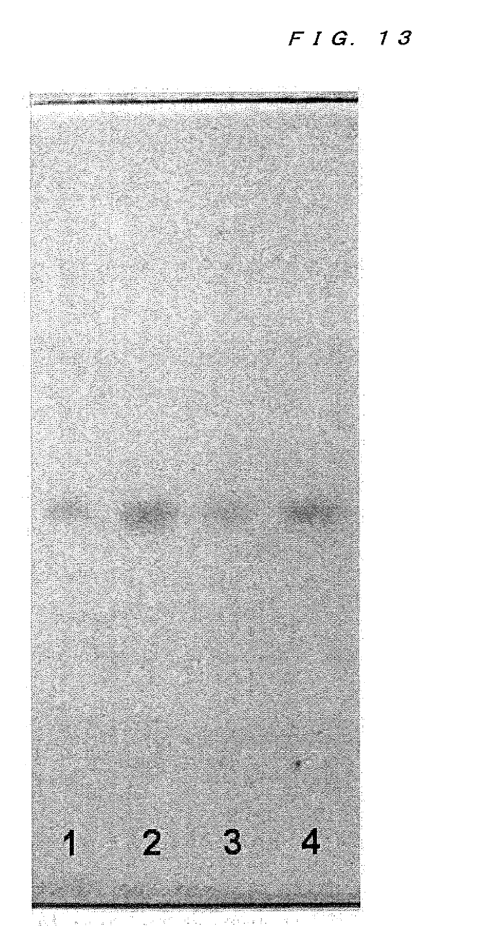

[FIG. 13] FIG. 13 is an image showing the presence of sialic acid-containing compounds detected by the resorcinol hydrochloric acid treatment after crudely extracting from cooked rice excluding a brown rice outermost layer part and including the remaining germ and aleurone layer part with water as the extraction solvent, and separating the resultant crude extract solution by the thin layer chromatography in Test Example 13.

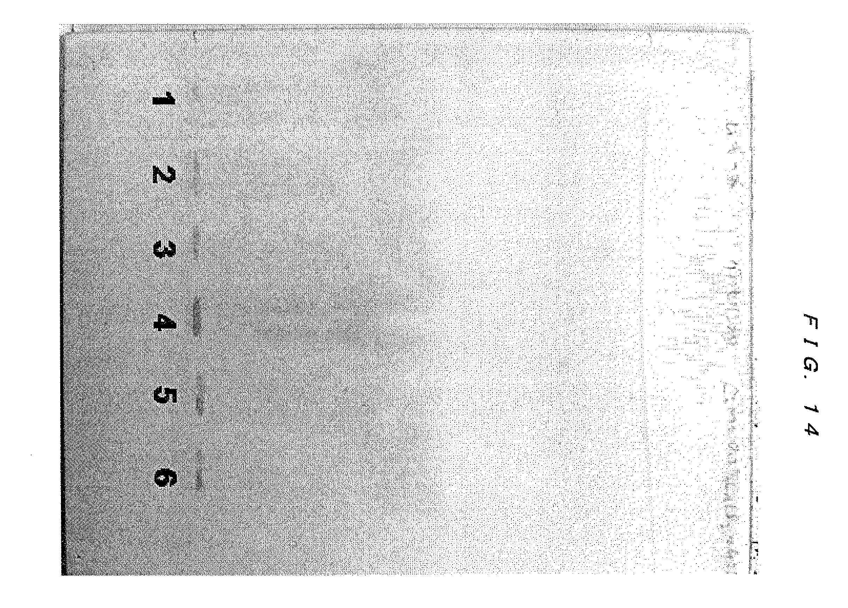

[FIG. 14] FIG. 14 is an image showing the presence of sialic acid-containing compounds detected by the resorcinol hydrochloric acid treatment after crudely extracting from wheat whole grain flour or barley whole grain flour with glycerol as the extraction solvent, and separating the resultant crude extract solution by the thin layer chromatography in Test Example 14.

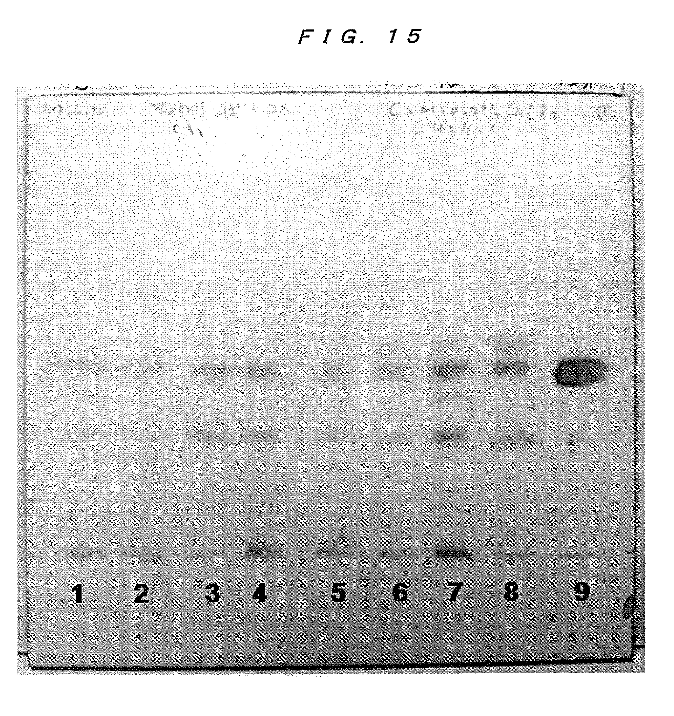

[FIG. 15] FIG. 15 is an image showing the presence of sialic acid-containing compounds detected by the resorcinol hydrochloric acid treatment after crudely extracting from wheat whole grain flour, barley whole grain flour, wheat bran, barley bran, or the rice germ with water as the extraction solvent, and separating the resultant crude extract solution by the thin layer chromatography in Test Example 15.

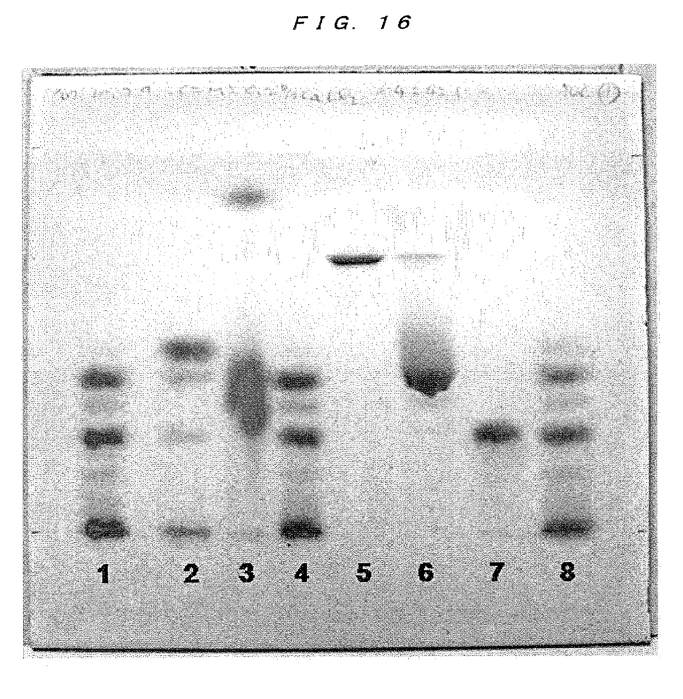

[FIG. 16] FIG. 16 is an image showing the presence of sialic acid-containing compounds detected by the resorcinol hydrochloric acid treatment after crudely extracting from wheat short with water, 11.83% acetic acid, or 7.06% hydrochloric acid as the extraction solvent, and separating the resultant crude extract solution by the thin layer chromatography in Test Example 16.

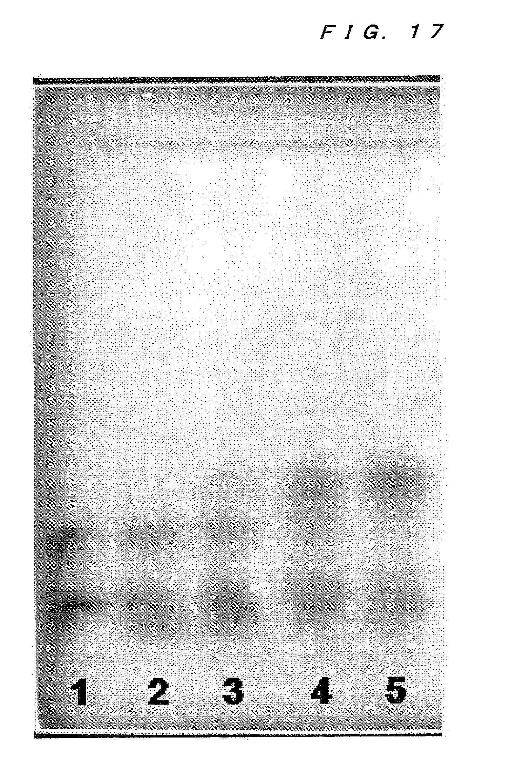

[FIG. 17] FIG. 17 is an image showing the presence of sialic acid-containing compounds detected by the resorcinol hydrochloric acid treatment after crudely extracting from wheat short with water as the extraction solvent, and separating the resultant crude extract solution by the thin layer chromatography in Test Example 17.

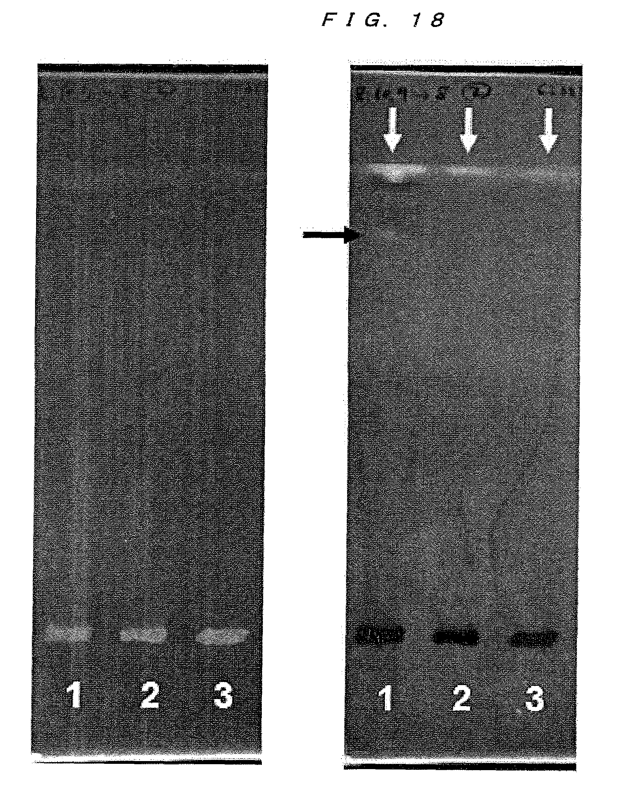

[FIG. 18] FIG. 18 is an image showing the presence of sialic acid-containing compounds detected by the lectin binding reaction after crudely extracting from rice bran, wheat bran, or barley bran with water as the extraction solvent, and separating the resultant crude extract solution by the thin layer chromatography in Test Example 18.

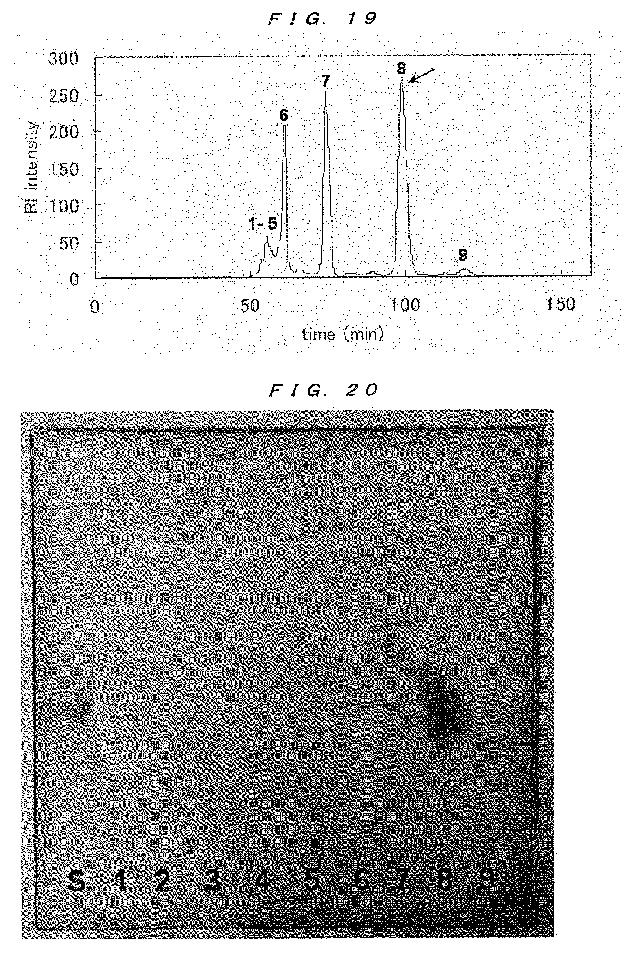

[FIG. 19] FIG. 19 is a chart in the case of extracting from rice whole grain flour with methanol, and fractionating the resultant extract solution with a gel filtration column in Example 4.
[FIG. 20] FIG. 20 is an image showing the presence of sialic acid-containing compounds detected by the resorcinol hydrochloric acid treatment after separating the fraction fractionated with the gel filtration column by the thin layer chromatography in Example 4.

### Description of Embodiments

Hereinafter, the present invention is described in detail.
The present invention relates to an extraction method for a sialic acid-containing compound, the method including: crudely extracting a soluble component from a raw material derived from a plant (in particular, seeds of cereal or seeds of bean) with water or an alcohol; and purifying the resultant crude extract solution.

### <Sialic acid-containing compound>

The "sialic acid-containing compound" in the present invention refers to a compound having sialic acid in its molecular structure. Sialic acid is a generic term for acyl derivatives of neuraminic acid that is an amino sugar, and 20 or more types of derivatives are available. Naturally, sialic acid is present as N-acetylneuraminic acid and N-glycolylneuraminic acid in the largest amounts. As represented by the structure of N-acetylneuraminic acid of the following chemical formula (1), sialic acid is a nonose having a skeleton comprising nine carbon atoms, and is characterized by having a carboxyl group and an N-acyl group as functional groups.
Note that because of the discovery of 2-keto-3-deoxy-D-glycero-D-galacto-nononic acid (KDN) in which the aminoacyl group at the position 5 has been replaced by a hydroxyl group in 1986, a sugar having an α-keto acid of a nonose skeleton (2-keto-3-deoxynononic acid) is currently employed as the definition of sialic acid.

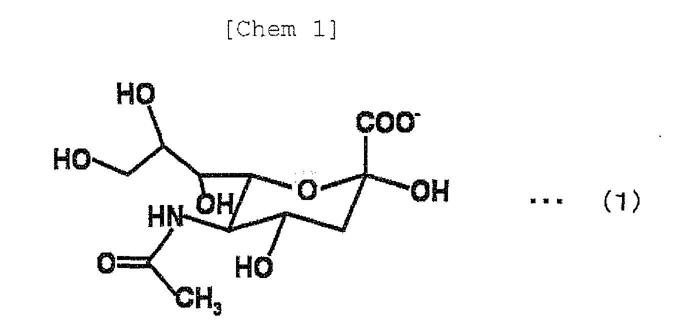

There are given, as molecular species corresponding to the sialic acid-containing compound, a polysialic acid sugar chain which is a 'sialic acid-containing sugar chain', fetuin, α1-acid glycoprotein and mucin which are 'sialic acid-containing glycoproteins containing the sugar chain', and ganglioside (sphingoglycolipid) which is a 'sialic acid-containing glycolipids'.
Here, polysialic acid is a sugar chain in which several tens or more of sialic acids are bound to each other. In particular, polysialic acid composes a sugar chain moiety of a neural cell adhesion molecule (NCAM) that is a huge nerve specific glycoprotein that functions in the fields of neural cell migration, neurite elongation, and synapse formation.
Further, ganglioside is a generic term for glycolipids each having sialic acid at the non-reducing terminal side, and a plurality of types thereof are available depending on the number and binding sites of sialic acids. Note that Examples described later suggest that most of sialic acid-containing compounds derived from plants in the present invention are glycolipids and glycoproteins or particularly glycolipids.
Further, sialic acid has negative charge, is involved in cell adhesion, cell differentiation, and the like, and in particular, is a substance essential for the differentiation, growth, and maintenance of neural cells.
Note that the sialic acid-containing compound in the present invention is a concept encompassing free sialic acid.

### <Raw material>

Any raw material may be used for the extraction of the sialic acid-containing compound in the present invention as long as the raw material is a plant body, i.e., a plant organ or tissue. Of those, it is preferred to use seeds of cereal and/or seeds of bean.
Here, the "cereals" refer to plants bearing edible starch-containing seeds among gramineous plants, and encompass types classified into miscellaneous cereals as well as wheat and rice.
Specific examples of the cereals include plants belonging to the family *Gramineae* such as rice, wheat, barley, rye, oats (cultivated species of oat and common wild oat), wild grass, finger millet, common millet, barnyard millet, foxtail millet, Job's tear, corn, sorghum (great millet, kaoliang, or sorghum), pearl millet, teff, fonio, kodra (kodo millet), and Indian rice.
In the present invention, of those, "bailey", "wheat", "rice", and "corn" as major cereals may be suitably used. Further, rye and oats closely related to wheat and barley may also be suitably used.

With regard to the 'barley', any cultivar and strain of plants belonging to *Hordeum vulgare* may be used. Examples of the barley include two-rowed barley, six-rowed barley, naked barlay, and hulled barley.
Further, with regard to the 'wheat', any species, cultivar, and strain of plants belonging to the genus Triticum may be used. Examples of the wheat include T. aestivum (common wheat and bread wheat), T. compactum (club wheat and compact ear wheat), and T. durum (durum wheat or macaroni wheat).
Further, with regard to the 'rice', any cultivar and strain of plants belonging to Oryza sativa, including Japonica cultivar and indica cultivar, may be used. Examples of the rice include common good-taste rice (such as Koshihikari or Hitomebore), slow-amylase rice, high-amylose rice, low-glutelin rice, glutinous rice, black rice, red rice, and purple rice.
Further, with regard to the 'corn' (maize), any cultivar and strain of plants belonging to *Zea* mays may be used. Examples of the corn include sweet corn (sweet taste species), popcorn (raw material for popcorn), dent corn (raw material for corn starch), flint corn, and waxy corn.

Further, with regard to the 'beans', there are given edible species belonging to the family *Leguminosae*. Examples of the beans include soybeans, adzuki beans, mung beans, kidney beans, peas, and field beans. Of those, soybeans and adzuki beans may be particularly suitably used.
Further, with regard to the 'soybeans' (*glycine max*), any cultivar and strain of plants belonging to Glycine max may be used. Examples of the soybeans include Kuro Mame, Aka Mame, Dadacha Mame, Ao Daizu, Shiro daizu, Gangui Mame, Miyagishirome, Oojiro, and Natto Kotsubu.
Further, with regard to the 'adzuki beans', any cultivar and strain of plants belonging to *Vigna angularis* may be used. Examples of the adzuki beans include Dainagon, Chunagon, Shiro Azuki, and Kuro Azuki.

Any cereals or beans may be used as the raw materials of the present invention as long as the cereals or beans are each a plant body, i.e., a plant organ or tissue. Of those, 'seeds' are preferably used.
Further, as long as the tissue is derived from seeds, any tissue or part may be used. For example, an embryo, germ, endosperm, seed coat, bran, aleurone layer, starch storage part, aleurone layer, or cotyledon may be used. In addition, the entire seeds (whole grains) may also be used.

Further, a processed product may be used as the raw material of the present invention. Examples of the processed product which may be used include wheat flour, barley flour, rice flour (such as brown rice flour or polished rice flour), cooked rice, corn flour, soybean flour, adzuki bean flour, and coconut powder.
In addition, wastes including bran (such as rice bran, wheat bran, barley bran, or germ) generated in a rice milling or polishing process, residue after the extraction of starch, shochu distillery by-products, waste (residue) with making soybean product (such as a byproduct of tofu (soybean card)-making), and irregular or excess crops may also be suitably used.

### <Crude extraction step>

The crude extraction of the sialic acid-containing compound in the present invention may be performed by the following method.
The above-mentioned raw material may be directly used in an extraction step depending on the type, tissue, or processed state of the raw material to be used. However, in view of an improvement in extraction efficiency, the raw material is desirably subjected to a treatment such as cutting finely or crushing or preferably a treatment such as milling, grinding, trituration, or pulverization (or particularly preferably pulverization) before the extraction step.
Note that each of those treatments may be performed by any method of known arts. For example, when seeds of cereals are pulverized, the seeds of cereals may be pulverized with a mill (Ultracentrifugal Mill manufactured by MRK-RETSCH or Cyclone Sample Mill manufactured by UDY CORPORATION), a brush type rice milling machine (HRG-122 manufactured by MINORU INDUSTRIAL Co., Ltd.), or a grinding type rice milling machine (Grain Testing Mill manufactured by Satake). Further, when beans are pulverized, a mill (Ultracentrifugal Mill manufactured by MRK-RETSCH) may be used.
Note that rice germ which is dropped out in a rice milling process and powdery processed products such as wheat flour and rice flour may be directly used for crude extraction without being subjected to milling or the like.

In this step, the above-mentioned material is immersed in a solvent to carry out a crude extraction treatment. With regard to a solvent used for the crude extraction treatment, any solvent may be used as long as the solvent is water, an alcohol, or an aqueous alcohol.
In this step, any molecular species of the sialic acid-containing compound can be extracted as long as the compound is soluble in those solvents (in particular, water). Further, some molecular species which have small water solubility or show different solubility depending on a change in pH may also be extracted by incorporating a salt, an acid, an alkali, saccharides, or a pH buffer into water to enhance the solubility.

In this step, the sialic acid-containing compound can be obtained from the above-mentioned raw material by simply using "water" as the solvent.
Here, any water such as tap water, distilled water, deionized water, or high hardness water may be used.

Further, examples of the "salt" include sodium chloride and sodium citrate. The salt may be incorporated to enhance the solubility of active components, to thereby enhance extraction efficiency.
Further, examples of the "acid" include hydrochloric acid, sulfuric acid, phosphoric acid, nitric acid, acetic acid, citric acid, and malic acid. Of those, hydrochloric acid and acetic acid may be suitably used. Note that the acid may be incorporated to reduce the molecular weight of the sialic acid-containing compound, to thereby enhance elution efficiency. Further, the acid provides an effect of inhibiting the growth of various kinds of harmful bacteria during long-term immersion at temperatures ranging from 25 to 40°C.
Further, examples of the "alkali" include sodium hydroxide, potassium hydroxide, calcium hydroxide, magnesium hydroxide, and ammonia. Of those, magnesium hydroxide and ammonia may be suitably used. The alkali provides an effect of inhibiting the growth of various kinds of harmful bacteria during long-term immersion at temperatures ranging from 25 to 40°C.
Further, examples of the "saccharides" include sorbitol, glucose, sucrose, trehalose, and xylitol.

In this step, the concentration of the salt, acid, alkali, saccharides, pH buffer, or the like to be incorporated into water varies depending on substances to be used. For example, in the case of using sodium chloride, it is desirable to incorporate sodium chloride at a concentration of 0.1 to 5% (w/v). Further, in the case of using hydrochloric acid, it is desirable to incorporate hydrochloric acid at a concentration of 0.5 to 12% (v/v) or preferably about 1% (v/v) to 7% (v/v) (about 0.28 N to 2N). Further, in the case of using acetic acid, it is desirable to incorporate acetic acid at a concentration of 0.5 to 12% (v/v) or preferably about 1.7% (v/v) to 12% (v/v) (about 0.28 N to 2 N).

Further, an "aqueous alcohol" or a "(100%) alcohol" may be used as the solvent in this step. Note that common water (such as tap water, distilled water, or deionized water) is used as water to be mixed into the aqueous alcohol, and water containing the salt, acid, alkali, saccharides, pH buffer, or the like described above may also be used.

Here, examples of the alcohol include: monoalcohols (monohydric alcohols) such as methanol, ethanol, butanol, and isopropanol; glycols (dihydric alcohols) such as ethylene glycol, propylene glycol, and polyethylene glycol; and glycerins (trihydric alcohols) such as glycerin and polyglycerin.
It is desirable to use, as the alcohol to be used in the present invention, methanol, ethanol, and isopropanol as monoalcohols (or more particularly methanol and ethanol as high-polar lower alcohols having less carbon atoms) among the above-mentioned alcohols. Moreover, it is particularly desirable to use ethanol in view of safety upon ingestion.
Further, ethylene glycol as one of glycols and glycerin as one of glycerins may also be suitably used.

In the solvent to be used in this crude extraction step, the concentration of the alcohol to water varies depending on the alcohol to be used. For example, in the case of using ethanol, it is desirable to incorporate ethanol at a concentration of 5% (v/v) to 100% (v/v: water content of 0%) or preferably 5% (v/v) to 50% (v/v).
Further, in the case of using ethylene glycol, it is desirable to incorporate ethylene glycol at a concentration of 20% (v/v) to 100% (v/v: water content of 0%) or preferably 50% (v/v) to 100% (v/v: water content of 0%).
The use of the solvent having a predetermined alcohol concentration is expected to provide effects of enhancing extraction efficiency and reducing contaminants.

In this crude extraction step, immersion maybe performed by adding 1 to 10 parts by mass or preferably 3 to 5 parts by mass of the above-mentioned solvent with respect to 1 part by mass of the above-mentioned raw material, to thereby extract active components. In addition, the raw material may also be subjected to ultrasonication or the like so that the raw material is crushed into a finer state, to thereby enhance extraction efficiency. For example, the ultrasonication may be performed by any conventionally employed method. For example, a B-42J Ultrasonic Cleaner (manufactured by BRANSON) may be used.

After that, a crude extraction treatment is performed at a temperature of 1 to 80°C or preferably 5 to 40°C for 1 minute to 24 hours or preferably 5 minutes to 12 hours.
Note that, when one wishes to enhance extraction efficiency, it is desirable to perform the crude extraction treatment for at least one hour or more. Further, when one wishes to prevent changes of active components due to an enzymatic metabolism, it is desirable to perform the crude extraction treatment at a temperature of 10°C or less (in particular, for 5 minutes to 4 hours).

Further, conversely, in order to enhance extraction efficiency by the reduction in molecular weight or fragmentation of the sialic acid-containing compound (enhancement in easily-soluble property), an endogenous enzyme contained in a plant body as a raw material can act on by performing the crude extraction treatment at about room temperature (for example, about 20 to 35°C) for 4 hours or more, preferably 12 hours or more, or more preferably 24 hours or more.
Further, (in particular, when a processed product of a plant body having an inactivated endogenous enzyme is used as a raw material), the reduction in molecular weight or fragmentation can be achieved by performing the crude extraction treatment in water containing a high concentration acid (for example, about 2 N hydrochloric acid or acetic acid) at about 40 to 80°C or preferably about 80°C.

In the crude extraction treatment, the elution efficiency can be enhanced by mixing, stirring, shaking, pressurization, or the like. (Note that, in particular, in the case of using the alcohol as the extraction solvent, the extraction may be performed sufficiently by simple stirring and mixing.)
Further, it is desirable to remove residues and impurities in the raw material by filtration, centrifugation, sedimentation, or the like after the crude extraction treatment.
In addition, in the case of using water (or water containing a salt, an acid, an alkali, saccharides, or a pH buffer) as the extraction solvent, an appropriate amount (for example, a 0.5 to 5-fold amount) of ethanol may be added to the crude extract solution, to thereby precipitate and remove high-molecular-weight contaminants such as nucleic acids and polysaccharides.

### <Purification step>

The content of the sialic acid-containing compound can be increased by purifying the crude extract solution obtained in the above-mentioned step. To be specific, the content can be increased by separating and recovering a fraction containing the sialic acid-containing compound in a large amount through dialysis, salt precipitation, or column chromatography.
In this step, the 'dialysis' may be performed by electrodialysis, simple dialysis, or the like.
Further, the 'salt precipitation' may be performed with sodium chloride, sodium sulfate, or the like.
Further, the 'column chromatography' may be performed with an affinity column (such as a serotonin affinity column, a lectin affinity column, a heparin column, or an antibody column), an ion-exchange column (such as a quaternary ammonium group-bound support column or a diethylaminoethyl group-bound support column), a gel filtration column (such as a cross-linked agarose gel column or a cross-linked dextran gel column), an adsorption resin column (such as a porous adsorption resin column or an active carbon column), a silica gel column, a modification group-bound silica gel column, a solid phase, or the like.
Further, with regard to the form of the column, a disposable column such as a solid-phase cartridge as well as a form used for high performance chromatography may be used without causing any problem.

Of those, an affinity column or a gel filtration column (in particular, an affinity column) is suitable from the viewpoint of an increase in content. Further, an adsorption resin column, a silica gel column, a dialysis method, and the like are given from the viewpoints of industrial practicability.

In particular, the 'affinity column' is preferably an affinity column packed with a column support showing strong specific affinity to the sialic acid-containing compound. In particular, the affinity column is suitably a serotonin affinity column or a lectin affinity column (or most suitably a serotonin affinity column).
To be specific, a serotonin affinity support (LAS-Serotonin supportmanufactured by J-OIL MILLS) may be used. Alternatively, a serotonin affinity support prepared by chemically coupling serotonin as a ligand with an appropriate commercially available support (for example, Sephadex manufactured by Pharmacia) may be used.
Note that a support capable of binding to sialic acid is, for example, a concanavalin A (cony) support, but the ConA support is not so suitable for the column support to be used in this step because the support utilizes the affinity to mannose to recover a sialic acid sugar chain containing mannose.
Further, a commercially-available, generally-used gel such as an agarose gel, a cross-linked agarose gel, or an acrylamide-based polymer gel may be used for the "gel filtration column".

The purity of the sialic acid-containing compound may be enhanced by employing those purification methods in combination. Further, the resultant purified product may be further subjected to HPLC to fractionate a peak containing the sialic acid-containing compound, to thereby further enhance the purity of the purified product and isolate a pure product.
Note that a fraction (purified product) containing the sialic acid-containing compound at a high concentration recovered through the above-mentioned purification is desirably used after the volatilization of an elution solvent or the like, dialysis removal, concentration to dryness, and the like.

The amount of the sialic acid-containing compound recovered through the above-mentioned purification step is about 2.5 to 4.5 mg in the case of extracting from 3 g of seeds of cereal or seeds of bean as the raw material with water, and purifying the extract with a serotonin affinity column, for example. Further, the purification can reduce the content of contaminants to a value equal to or less than the detection limit of the total amount of organic compounds detected under an iodine vapor atmosphere.

### <Applications>

The sialic acid-containing compound is a substance essential for the differentiation, growth, and maintenance of neural cells, and is expected to serve as an active component having therapeutic and prophylactic effects on: central nervous system disorders such as Alzheimer's disease, dementia, and spinal cord injury; and motor and sensory disorders associated with diabetic neuropathy and peripheral nerve diseases. Further, the compound can be expected to exert a memory-improving action in addition to the above-mentioned diseases.
In addition, the compound is also known to have an immunostimulatory action and an anticancer action.

Accordingly, the sialic acid-containing compound (purified product) derived from a plant through the above-mentioned steps may be mixed into various raw materials to produce a medicament, a functional food or beverage, a functional cosmetic, and the like.
Note that the extract serving as the active component in the present invention is derived from a plant (in particular, seeds of cereal or seeds of bean) and hence is excellent in safety upon administration and ingestion.

The form of the medicament maybe a powder, a fine granule, or a granule, for example, in the case of oral administration. In addition, the form may also be a capsule into which the medicament is packed, a solution in which the medicament is dispersed in water, or a tablet obtained by blending the medicament with an excipient and the like.
Further, in the case of transdermal administration, the form may be, for example, a cream, a gel, a seal, or a form in which a support containing an active component is fixed.

Further, in order to produce the functional food or beverage (food for specified health use, neutraceutical and functional food, or health food), the compound may be added to a variety of foods such as ham, sausage, steamed fish paste sausage, a hollow tube-like fish cake, bread, butter, powdered milk, and confectionery. Alternatively, the compound may be added to beverages such as water, alcoholic liquors, fruit juice, cow's milk, and soft drink.

In addition, with regard to the functional cosmetic, for example, the compound may be added to a form suitable for cosmetics such as a cream, a gel, a lotion, or hair dressing, to thereby produce a form suitable for transdermal administration.

### Examples

The present invention is described in more detail by examples, but the scope of the present invention is by no means limited by these examples.

### <Test Example 1> (Detection of sialic acid-containing compounds from crude extract solution of soybean seeds)

15 mL of water were added to 3 g of soybean flour obtained by milling whole soy (Sachiyutaka) with a mill (Ultracentrifugal Mill manufactured by MRK-RETSCH, mesh diameter: 0.5 mm or 1 mm), and the contents were mixed well (sample 1-1). The mixture was subjected to ultrasonication using an ultrasonic apparatus (B-42J Ultrasonic Cleaner manufactured by BRANSON) for 1 minute and 30 seconds. The treated product was left to stand still at 10°C for 12 hours and then centrifuged at 3000 rpm for 10 minutes to perform solid-liquid separation.
An equal amount of ethanol was added to the separated crude extract solution, and the contents were mixed well. After that, the mixture was left to stand still at 10°C for 12 hours and then centrifuged at 3000 rpm for 10 minutes to perform solid-liquid separation.
The separated supernatant was developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 1 shows the results.

### <Test Example 2> (Detection of sialic acid-containing compounds from crude extract solutions of brown rice whole grains and brown rice aleurone layer part)

15 mL of water were added to 3 g of flour obtained by milling brown rice (Koshihikari) whole grains with a mill (Cyclone Sample Mill manufactured by UDY Corporation), and the contents were mixed well (sample 2-2).
Further, 15 mL of water were added to 3 g of flour derived from a part in the range of 96 to 91% by mass corresponding to an aleurone layer part when brown rice was regarded as 100% by mass (brown rice aleurone layer part recovered by grinding out), the part being obtained by grinding out brown rice (Koshihikari) successively from the outside using a grinding type rice milling machine (Satake Grain Testing Mill manufactured by Satake), and the contents were mixed well (sample 2-5).
Those mixtures were subjected to ultrasonication using an ultrasonic apparatus (B-42J Ultrasonic Cleaner manufactured by BRANSON) for minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃0H/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 1 shows the results.

### <Test Example 3> (Detection of sialic acid-containing compounds from crude extract solution of wheat flour)

15 mL of water were added to 3 g of wheat flour (Norin 61), and the contents were mixed well (sample 3-3). 15 mL of water were added to 3 g of wheat flour (Western White), and the contents were mixed well (sample 3-4).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4 /4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 1 shows the results.

Note that, in FIG. 1, the crude extracts obtained using water as the extraction solvent in the above-mentioned steps were developed on a silica gel plate, wherein lane 1 shows that from the flour of whole soy (Sachiyutaka) (sample 1-1), lane 2 shows that from the brown rice (Koshihikari) whole grain flour (sample 2-2), lane 3 shows that from the wheat flour (Norin 61) (sample 3-3), lane 4 shows that from the wheat flour (Western White) (sample 3-4), and lane 5 shows that from the flour of the brown rice (Koshihikari) aleurone layer part (sample 2-5), respectively.

### [Results in Test Examples 1 to 3]

As shown in FIG. 1, the results revealed that the sialic acid-containing compound was contained in each of the crude extract solutions obtained in the above-mentioned steps using water as the extraction solvent and using the whole soy flour (sample 1-1) (lane 1), the brown rice whole grain flour (sample 2-2) (lane 2), the wheat flours (samples 3-3, 3-4) (lane 3, 4), and the flour of the brown rice aleurone layer part (sample 2-5) (lane 5) as the raw materials. It was also shown that the sialic acid-containing compound capable of being extracted by the above-mentioned method was contained particularly in abundance in each of the whole soy flour (sample 1-1) (lane 1) and the flour of the brown rice aleurone layer part (sample 2-5) (lane 5) among those samples.
The results revealed that the crude extract solution containing the sialic acid-containing compound was obtained from each of the flours derived from the seeds of the soybean, the rice, and the wheat by using water as the extraction solvent without using a harmful organic solvent.
Further, most of the detected sialic acid-containing compounds migrated greatly from the origin, which suggested that the compounds were glycolipids as substances each having high affinity to a developing solvent. Note that the sialic acid-containing compounds that did not migrate from the vicinity of the origin in the samples 1-3 and 1-4 (lanes 3 and 4) are estimated to be glycoproteins adsorbed onto the silica gel plate.

### <Test Example 4> (Detection of sialic acid-containing compounds from crude extract solution using ethylene glycol)

15 mL of 100% (v/v) ethylene glycol were added to 3 g of flour derived from a part in the range of 96 to 91% by mass corresponding to an aleurone layer part when brown rice was regarded as 100% by mass (brown rice aleurone layer part recovered by grinding out), the part being obtained by grinding out brown rice (Koshihikari) successively from the outside using a grinding type rice milling machine (Grain Testing Mill manufactured by SATAKE), and the contents were mixed well (sample 4-7). In addition, 15 mL of 100% (v/v) ethylene glycol were added to 3 g of rice germ (Koshihikari), and the contents were mixed well (sample 4-8).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
The separated supernatants were developed with CH₃Cl /CH₃OH/ 0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 2 shows the results.

Note that, in FIG. 2, lane 1 is authentic N-acetylneuraminic acid (containing sialic acid) (N-Acetylneuraminic acid Type IV-S, manufactured by SIGMA), lane 2 is authentic ganglioside G_{D1a} (containing sialic acid) (Disialoganglioside-G_{D1a}, manufactured by SIGMA), lane 3 is authentic ganglioside G_{T1b} (containing sialic acid) (Trisialoganglioside-G_{T1b}, manufactured by SIGMA), lane 4 is authentic phosphatidylserine (Phosphatidylserine from beef brain, manufactured by SERDANY Research Laboratories), lane 5 is authentic phosphatidylethanolamine (L-α-Phosphatidylethanolamine from plant, manufactured by AVANTI Polar-Lipids), and lane 6 is authentic lysophosphatidylcholine (L-α-Lysophosphatidylcholine from soybean, manufactured by SIGMA), which were respectively developed on the silica gel plate.
The result of development of the crude extract solution on a silica gel plate obtained in the above-mentioned step by using 100 % (v/v) ethylene glycol as the extraction solvent from the flour of the brown rice (Koshihikari) aleurone layer part (sample 4-7) is shown in lane 7 and that from the rice germ (Koshihikari) (sample 4-8) is shown in lane 8, respectively.

As shown in FIG. 2, the results demonstrated that the sialic acid-containing compound was also contained in the crude extract solution obtained in the above-mentioned steps by using 100% (v/v) ethylene glycol as the extraction solvent and using the flour of the brown rice aleurone layer part (sample 4-7) (lane 7) or the rice germ (sample 4-8) (lane 8) as the raw material.
It was also shown that the sialic acid-containing compound was contained in the rice germ part (sample 4-8) (lane 8) in abundance equal to the brown rice aleurone layer part (sample 4-7) (lane 7).
Further, the detected sialic acid-containing compounds migrated greatly from the origin, which suggested that the compounds were glycolipids as substances each having high affinity to a developing solvent.

### <Test Example 5> (Influence of ethylene glycol on extraction)

15 mL of water were added to 3 g of flour derived from a part in the range of 96 to 91% by mass corresponding to an aleurone layer part when brown rice was regarded as 100% by mass (brown rice aleurone layer part recovered by grinding out), the part being obtained by grinding out brown rice (Koshihikari) successively from the outside using a grinding type rice milling machine (Satake Grain Testing Millmanufactured by SATAKE), and the contents were mixed well (sample 5-1). In addition, 15 mL of 100% (v/v) ethylene glycol were added to 3 g of the flour of the brown rice aleurone layer part, and the contents were mixed well (sample 5-2).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (V/V/V) and separated into individual components on a silica gel plate.
The silica gel plate on which the supernatants had been developed was left to stand still under an iodine vapor atmosphere at room temperature for 72 hours, to thereby detect all organic compounds. Subsequently, the silica gel plate taken out of the iodine vapor atmosphere was left to stand still for 24 hours to desorb iodine sufficiently, sprayed with resorcinol hydrochloric acid, and heated at 95°C to develop a color, to thereby detect sialic acid-containing compounds. FIGS. 3 show the results. Note that, in FIGS. 3, FIG. 3 (a) shows the all organic compounds detected and FIG. 3(b) shows the sialic acid-containing compounds detected.

Note that, in FIG. 3, each lane shows a development on a slica gel plate, wherein lane 1 shows that of the crude extract solution (sample 5-1) obtained from the flour of the brown rice aleurone layer part using water as the extraction solvent in the above-mentioned steps and lane 2 shows that of the crude extract solution (sample 5-2) obtained from the flour of the brown rice aleurone layer part using 100% (v/v) ethylene glycol as the extraction solvent in the above-mentioned steps.

The results of the comparison between FIG. 3 (a) and FIG. 3 (b) showed that the extraction (sample 5-2) (lane 2) of the flour of the brown rice aleurone layer part using 100% (v/v) ethylene glycol as the extraction solvent provided a crude extract solution containing the sialic acid-containing compound and less contaminants than the extraction (sample 5-1) (lane 1) using water as the extraction solvent.
Further, the detected sialic acid-containing compounds migrated greatly from the origin, which suggested that the compounds were glycolipids as substances each having high affinity to a developing solvent.

### <Test Example 6> (Influence of ethanol precipitation treatment on extraction)

15 mL of water were added to 3 g of flour derived from a part in the range of 96 to 91% by mass corresponding to an aleurone layer part when brown rice was regarded as 100% by mass (brown rice aleurone layer part recovered by grinding out), the part being obtained by grinding out brown rice (Koshihikari) successively from the outside using a grinding type rice milling machine (Grain Testing Mill manufactured by STAKE), and the contents were mixed well (samples 6-1 to 6-3). Further, 15 mL of water were added to 3 g of flour derived from a part (polished rice surface layer part recovered by grinding out) in the range of 91 to 86% by mass corresponding to a polished rice surface layer part when brown rice was regarded as 100% by mass, the part being obtained by grinding out brown rice (Koshihikari) successively from the outside using the grinding type rice milling machine (Grain Testing Mill manufactured by STAKE), and the contents were mixed well (samples 6-4 to 6-6).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.

Concerning the samples obtained in the above-mentioned steps from the flour of the brown rice aleurone layer part and from the flour of the polished rice surface layer part, "centrifuged supernatants after the addition of ethanol" were designated as the samples 6-1 and 6-4, "solutions obtained by redissolving centrifuged precipitates in water after the addition of ethanol" were designated as the samples 6-2 and 6-5, and "solutions obtained by redissolving centrifuged precipitates in water after the first crude extraction with water" were designated as the samples 6-3 and 6-6, and those samples were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 4 shows the results.

Note that, in FIG. 4, each lane shows a development on a slica gel plate, wherein lane 1 shows that of the centrifuged supernatant after the addition of ethanol obtained from the flour of the brown rice aleurone layer part (sample 6-1), lane 2 shows that of the solution obtained from the flour of the brown rice aleurone layer part by redissolving the centrifuged precipitate in water after the addition of ethanol (sample 6-2), lane 3 shows that of the solution obtained from the flour of the brown rice aleurone layer part by redissolving the centrifuged precipitate in water after the first crude extraction with water (sample 6-3), lane 4 shows that of the centrifuged supernatant after the addition of ethanol obtained from the flour of the polished rice surface layer part (sample 6-4), lane 5 shows that of the solution obtained from the flour of the polished rice surface layer part by redissolving the centrifuged precipitate in water after the addition of ethanol (sample 6-5), and lane 6 shows that of the solution obtained from the flour of the polished rice surface layer part by redissolving the centrifuged precipitate in water after the first crude extraction with water (sample 6-6), respectively.
Further, lane 7 is authentic ganglioside G_{D1a} (containing sialic acid) (Disialoganglioside-G_{D1a}, manufactured by SIGMA), lane 8 is authentic ganglioside G_{T1b} (containing sialic acid) (Trisialoganglioside-G_{T1b}, manufactured by SIGMA), and lane 9 is authentic N-acetylneuraminic acid (containing sialic acid) (N-Acetylneuraminic acid Type IV-S, manufactured by SIGMA), which were respectively developed on the silica gel plate.

As shown in FIG. 4, the results demonstrated that the sialic acid-containing compound was contained particularly in abundance in each of the centrifuged supernatants (crude extract solution) (samples 6-1, 6-4) (lanes 1 and 4) after the addition of ethanol obtained from the flour of the brown rice aleurone layer part and the polished rice surface layer part by the above-mentioned extraction method.
In addition, when the flour of the brown rice aleurone layer part was used as the raw material, it was also shown that the sialic acid-containing compound was contained in abundance in the solution obtained by redissolving the centrifuged precipitate in water after the addition of ethanol (sample 6-2) (lane 2).
Note that the sialic acid-containing compound was scarcely contained in each of the solutions (samples 6-3 and 6-6) (lanes 3 and 6) obtained from the flour of the brown rice aleurone layer part and the polished rice surface layer part by redissolving the centrifuged precipitate (residue) in water after the first crude extraction with water, and thus, it was shown that most of the sialic acid-containing compound had been extracted into the crude extract solution in the first crude extraction with water.
Further, most of the detected sialic acid-containing compounds migrated greatly from the origin, which suggested that the compounds were glycolipids as substances each having high affinity to a developing solvent. Note that the sialic acid-containing compounds that did not migrate from the vicinity of the origin in the samples 6-1 and 6-4 (lanes 1 and 4) are estimated to be glycoproteins adsorbed onto the silica gel plate.

### <Test Example 7> (Detection of sialic acid-containing compounds from crude extract solutions using various solvents)

15 mL of water were added to 3 g of flour obtained by milling adzuki bean seeds with a mill (Ultracentrifugal Mill manufactured by MRK-RETSCH), and the contents were mixed well (samples 7-1, 7-4, and 7-7). In addition, 15 mL of 1% (v/v) hydrochloric acid were added to 3 g of the adzuki bean seed flour, and the contents were mixed well (samples 7-2, 7-5, and 7-8). In addition, 15 mL of 100% (v/v) ethylene glycol were added to 3 g of the adzuki bean seed flour, and the contents were mixed well (samples 7-3, 7-6, and 7-9) .
Those mixtures were subjected to ultrasonication using an ultrasonic apparatus (B-42J Ultrasonic Cleaner manufactured by BRANSON) for 1 minute and 30 seconds, Those treated products were left to stand still at 10°C (samples 7-7 to 7-9), 25°C (samples 7-4 to 7-6), or 37°C (samples 7-1 to 7-3) for 12 hours, and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (v/v/v) and separated into individual components on a silica gel plate. The silica gel plate was sprayed with resorcinol hydrochloric acid and heated at 95°C to develop a color, to thereby detect sialic acid-containing compounds. FIG. 5 shows the results.

Note that, in FIG. 5, each lane shows a development on a slica gel plate, wherein lane 1 shows that of the crude extract solution obtained from the adzuki bean seed flour using water at 37°C as the extraction solvent in the above-mentioned steps (sample 7-1), lane 2 shows that of the crude extract solution obtained from the adzuki bean seed flour using 1% (v/v) hydrochloric acid at 37°C as the extraction solvent in the above-mentioned steps (sample 7-2), lane 3 shows that of the crude extract solution obtained from the adzuki bean seed flour using 100% (v/v) ethylene glycol at 37°C as the extraction solvent in the above-mentioned steps (sample 7-3), lane 4 shows that of the crude extract solution obtained from the adzuki bean seed flour using water at 25°C as the extraction solvent in the above-mentioned steps (sample 7-4), lane 5 shows that of the crude extract solution obtained from the adzuki bean seed flour using 1% (v/v) hydrochloric acid at 25°C as the extraction solvent in the above-mentioned steps (sample 7-5), lane 6 shows that of the crude extract solution obtained from the adzuki bean seed flour using 100% (v/v) ethylene glycol at 25°C as the extraction solvent in the above-mentioned steps (sample 7-6), lane 7 shows that of the crude extract solution obtained from the adzuki bean seed flour using water at 10°C as the extraction solvent in the above-mentioned steps (sample 7-7), lane 8 shows that of the crude extract solution obtained from the adzuki bean seed flour using 1% (v/v) hydrochloric acid at 10 °C as the extraction solvent in the above-mentioned steps (sample 7-8), and lane 9 shows that of the crude extract solution obtained from the adzuki bean seed flour using 100% (v/v) ethylene glycol at 10°C as the extraction solvent in the above-mentioned steps (sample 7-9), respectively.

As shown in FIG. 5, the results demonstrated that the crude extract solution containing the sialic acid-containing compound was also obtained from the flour of the adzuki bean seeds by extraction using water, 1% (v/v) hydrochloric acid, or 100% (v/v) ethylene glycol as the extraction solvent at a temperature of 10, 25, or 37°C.
Further, the detected sialic acid-containing compounds migrated greatly from the origin, which suggested that the compounds were glycolipids as substances each having high affinity to a developing solvent.

### <Test Example 8> (Detection of sialic acid-containing compounds from crude extract solution of each layer of brown rice)

15 mL of water were added to 3 g of flour derived from a part in the range of 100 to 96% by mass corresponding to an outermost layer part when brown rice was regarded as 100% by mass (brown rice outermost layer part recovered by grinding out), the part being obtained by grinding out brown rice (Koshihikari) successively from the outside using a grinding type rice milling machine (Grain Testing Mill manufactured by SATAKE), and the contents were mixed well (sample 8-1). Further, 15 mL of water was added to 3 g of flour derived from a part (brown rice aleurone layer part recovered by grinding out) in the range of 96 to 91% by mass corresponding to an aleurone layer part when brown rice was regarded as 100% by mass, the part being obtained by grinding out brown rice (Koshihikari) successively from the outside using the grinding type rice milling machine (Grain Testing Mill manufactured by STAKE), and the contents were mixed well (sample 8-2). In addition, 15 mL of water were added to 3 g of flour derived from a part in the range of 91 to 86% by mass corresponding to a polished rice surface layer part when brown rice was regarded as 100% by mass (polished rice surface layer part recovered by grinding out), the part being obtained by grinding out brown rice (Koshihikari) successively from the outside using the grinding type rice milling machine (Grain Testing Mill manufactured by SATAKE), and the contents were mixed well (sample 8-3).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 6 shows the results.

### <Test Example 9> (Detection of sialic acid-containing compounds from crude extract solution of dehulled black soybean seed cotyledon part)

15 mL of water were added to 3 g of flour obtained by milling a cotyledon part of dehulled black soybean seeds with a mill (Ultracentri-fugal Mill manufactured by MRK-RETSCH), and the contents were mixed well (sample 9-4). Further, 15 mL of 1% (v/v) hydrochloric acid were added to 3 g of the flour of the cotyledon part of dehulled black soybean seeds, and the contents were mixed well (sample 9-5).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 6 shows the results.

### <Test Example 10> (Detection of sialic acid-containing compounds from crude extract solution of corn seeds)

15 mL of water were added to 3 g of flour obtained by milling corn seeds (popcorn seeds) with a mill (Ultracentrifugal Mill manufactured by MRK-RETSCH), and the contents were mixed well (sample 10-6). Further, 15 mL of 1% (v/v) hydrochloric acid were added to 3 g of the flour of the corn seed (popcorn seeds), and the contents were mixed well (sample 10-7).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/0.2%, CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 6 shows the results.

Note that, in FIG. 6, each lane shows a development on a slica gel plate, wherein lane 1 shows that of the crude extract solution obtained from the flour of the brown rice outermost layer part using water as the extraction solvent in the above-mentioned steps (sample 8-1), lane 2 shows that of the crude extract solution obtained from the flour of the brown rice aleurone layer part using water as the extraction solvent in the above-mentioned steps (sample 8-2), lane 3 shows that of the crude extract solution obtained from the flour of the polished rice surface layer part using water as the extraction solvent in the above-mentioned steps (sample 8-3), lane 4 shows that of the crude extract solution obtained from the flour of the cotyledon part of the black soybean seed using water as the extraction solvent in the above-mentioned steps (sample 9-4), lane 5 shows that of the crude extract solution obtained from the flour of the cotyledon part of the black soybean seed using 1% (v/v) hydrochloric acid as the extraction solvent in the above-mentioned steps (sample 9-5), lane 6 shows that of the crude extract solution obtained from the corn seeds using water as the extraction solvent in the above-mentioned steps (sample 10-6), and lane 7 shows that of the crude extract solution obtained from the corn seeds using 1% (v/v) hydrochloric acid as the extraction solvent in the above-mentioned steps (sample 10-7), respectively.
Further, lane 8 is authentic Ganglioside G_{D1a} (containing sialic acid) (Disialoganglioside-G_{D1a}, manufactured by SIGMA) and lane 9 is authentic ganglioside G_{T1b} (containing sialic acid) (Trisialoganglioside-G_{T1b}, manufactured by SIGMA), which were respectively developed on the silica gel plate.

### [Results in Test Examples 8 to 10]

As shown in FIG. 6, the results demonstrated that the sialic acid-containing compound was also contained in the crude extract solution (sample 8-1) (lane 1) obtained from the flour of the brown rice outermost layer part using water as the extraction solvent in the above-mentioned steps in abundance equal to the crude extract solution (sample 8-2 or 8-3) (lane 2 or 3) obtained from the flour of the brown rice aleurone layer part or the flour of the polished rice surface layer part using water as the extraction solvent in the above-mentioned steps.
It was also shown that the sialic acid-containing compound was also contained in the crude extract solution (sample 9-4 or 9-5) (lane 4 or 5) obtained from the flour of the black soybean cotyledon using water or 1% (v/v) hydrochloric acid as the extraction solvent in the above-mentioned steps in abundance equal to the crude extract solution (sample 8-2 or 8-3) (lane 2 or 3) obtained from the flour of the brown rice aleurone layer part or the flour of the polished rice surface layer part using water as the extraction solvent in the above-mentioned steps.
It was also shown that the sialic acid-containing compound was also contained in the crude extract solution (sample 10-6 or 10-7) (lane 6 or 7) obtained from the flour of the corn using water or 1% (v/v) hydrochloric acid as the extraction solvent in the above-mentioned steps.
Further, the detected sialic acid-containing compounds migrated greatly from the origin. The fact suggested that the compounds were glycolipids as substances each having high affinity to a developing solvent.

### <Test Example 11> (Influence of extraction time on extraction)

15 mL of water were added to 3 g of the rice germ (Koshihikari), and the contents were mixed well (samples 11-1, 11-2, 11-5, and 11-6). Further, 15 mL of water were added to 3 g of flour derived from a part in range of 96 to 91% by mass corresponding to an aleurone layer part when brown rice was regarded as 100% by mass (brown rice aleurone layer part recovered by grinding out), the part being obtained by grinding out brown rice (Koshihikari) successively from the outside using a grinding type rice milling machine (Grain Testing Mill manufactured by SATAKE), and the contents were mixed well (samples 11-3, 11-4, 11-7, and 11-8).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 2.5 hours (samples 11-1 to 11-4) or 10 hours (samples 11-5 to 11-8) and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.

A "centrifuged supernatant after the addition of ethanol" obtained from the rice germ or from the flour of the brown rice aleurone layer part in the above-mentioned steps was designated as a sample 11-2, 11-4, 11-6, or 11-8, and a "centrifuged supernatant after the first crude extraction with water" was designated as a sample 11-1, 11-3, 11-5, or 11-7, and those samples were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 7 shows the results.

Note that, in FIG. 7, each lane shows a development on a slica gel plate, wherein lane 1 shows that of the centrifuged supernatant after the extraction from the rice germ with water for 2.5 hours (sample 11-1), lane 2 shows that of the centrifuged supernatant after the addition of ethanol obtained in the above-mentioned steps after the extraction from the rice germ with water for 2. 5 hours (sample 11-2), lane 3 shows that of the centrifuged supernatant after the extraction from the flour of the brown rice aleurone layer part with water for 2.5 hours (sample 11-3), lane 4 shows that of the centrifuged supernatant after the addition of ethanol obtained in the above-mentioned steps after the extraction from the flour of the brown rice aleurone layer part with water for 2.5 hours (sample 11-4), lane 5 shows that of the centrifuged supernatant after the extraction from the rice germ with water for 10 hours (sample 11-5), lane 6 shows that of the centrifuged supernatant after the addition of ethanol obtained in the above-mentioned steps after the extraction from the rice germ with water for 10 hours (sample 11-6), lane 7 shows that of the centrifuged supernatant after the extraction from the flour of the brown rice aleurone layer part with water for 10 hours (sample 11-7), and the lane 8 shows that of the centrifuged supernatant after the addition of ethanol obtained in the above-mentioned steps after the extraction from the flour of the brown rice aleurone layer part with water for 10 hours (sample 11-8) in lane 8, respectively.

As shown in FIG. 7, the results demonstrated that the sialic acid-containing compound was contained in abundance in the crude extract solution obtained by extracting from the rice germ or the flour of the brown rice aleurone layer part with water at 10°C for 2.5 or 10 hours. It was also shown that the amount of the sialic acid-containing compound capable of being extracted with water for 10 hours (samples 11-5 to 11-8) (lanes 5 to 8) was increased compared with the case of extraction for 2.5 hours (samples 11-1 to 11-4) (lanes 1 to 4).
Note that, no large difference in extracted amount of the sialic acid-containing compound was observed between the centrifuged supernatants after the extraction with water (samples 11-1, 11-3, 11-5, and 11-7) (lanes 1, 3, 5, and 7) and the centrifuged supernatants after the addition of ethanol (samples 11-2, 11-4, 11-6, and 11-8) (lanes 2, 4, 6, and 8).
Further, most of the detected sialic acid-containing compounds migrated greatly from the origin. The fact suggested that the compounds were glycolipids as substances each having high affinity to a developing solvent. Note that the sialic acid-containing compounds that did not migrate from the vicinity of the origin in the samples 11-3, 11-4, and 11-7 (lanes 3, 4, and 7) are estimated to be glycoproteins adsorbed onto the silica gel plate.

### <Example 1> (Extraction from brown rice aleurone layer part with water and affinity column purification).

### [Serotonin affinity column purification]

In the same manner as in Test Example 2, 15 mL of water were added to 3 g of flour derived from a part in the range of 96 to 91% by mass corresponding to an aleurone layer part when brown rice was regarded as 100% by mass (brown rice aleurone layer part recovered by grinding out), the part being obtained by grinding out brown rice (Koshihikari) successively from the outside using a grinding type rice milling machine (Grain Testing Mill manufactured by SATAKE), and the contents were mixed well.
The mixture was subjected to ultrasonication for 1 minute and 30 seconds. The treated product was left to stand still at 10°C for 12 hours and then centrifuged at 3000 rpm for 10 minutes to perform solid-liquid separation.
An equal amount of ethanol was added to the separated extract supernatant. The contents were mixed well, left to stand still at 10°C for 12 hours and then centrifuged at 3000 rpm for 10 minutes to perform solid-liquid separation.
1 mL of the separated crude extract solution was applied to a column with a diameter of 4 mm and a length of 5 cm (LAS-Serotonin manufactured by J-Oil Mills) packed with a serotonin affinity support (LAS-Serotonin Support manufactured by J-Oil Mills) and fractionated. The conditions of chromatography were as described below. Pure water was run at a flow rate of 0.2 mL/min for 90 minutes (zone 1) to remove unadsorbed components, and subsequently an ammonium acetate solution with a linear gradient from 4 mM to 30 mM was run for 150 minutes (zone 2) to recover the sialic acid-containing compound dissociated from the support. Finally, pure water was run for 90 minutes to regenerate the column (zone 3).
The sialic acid-containing compound was detected by measuring the amount of the compound eluted from the serotonin affinity support in terms of an absorbance at an OD 280 nm. The results are shown in FIG. 8.

Further, the fractionation with the serotonin affinity column was performed twice (twice using 1 mL of the crude extract solution each time). Fractions (zone 2) in which the sialic acid-containing compound had been eluted in twice of the fractionation were freeze-dried with a freeze dryer (Freezdryer FD-1 manufactured by EYELA), and the weights of the dried products were measured.

As shown in FIG. 8, the results demonstrated that the recovery of the fractions in which the sialic acid-containing compound dissociated from the serotonin affinity column had been eluted was attained by running the ammonium acetate solution with a linear gradient from 4 mM to 30 mM through the column (zone 2), after running pure water through the column to remove the component unadsorbed to the serotonin affinity support from the column (zone 1). It was also shown that the sialic acid-containing compound was particularly abundantly eluted in the fractions at around 130 to 160 minutes after the start of elution (start of zone 1) in the zone 2.
In addition, from the fractions (zone 2) obtained by fractionating 2 mL of the crude extract solution (twice by 1 mL) with the serotonin affinity column, 0.5 mg of a dry solid was obtained, and thus, it was shown that 5 mg of the dry solid of the sialic acid-containing compound were obtained from 3 g of the flour of the brown rice aleurone layer part as the raw material (20 mL of the crude extract solution were obtained from 3 g of the flour of the brown rice aleurone layer part through the above-mentioned steps).

### <Example 2> (Extraction from rice germ with water and purification)

### [Serotonin affinity column purification]

15 mL of water were added to 3 g of rice germ (Koshihikari), and the contents were mixed well. The mixture was subjected to ultrasonication for 1 minute and 30 seconds. The treated product was left to stand still at 10°C for 12 hours and then centrifuged at 3000 rpm for 10 minutes to perform solid-liquid separation.
The separated crude extract solution (0.1 mL) was applied to the column with a diameter of 4 mm and a length of 5 cm (LAS-Serotonin manufactured by J-Oil Mills) packed with the serotonin affinity support (LAS-Serotonin Support manufactured by J-Oil Mills), and fractionated. The conditions of chromatography were as described below. Pure water was run at a flow rate of 0.2 mL/min for 285 minutes to remove the unadsorbed components, and subsequently running of pure water was stopped for 12 hours in order to assure the interaction between the sialic acid-containing compound and the serotonin affinity support. Subsequently, a 30 mM ammonium acetate solution was run for 285 minutes to recover the sialic acid-containing compound dissociated from the support (note that, regeneration of the column was omitted in this example).
The sialic acid-containing compound was detected by measuring the amount of the compound eluted from the serotonin affinity support in terms of an absorbance at OD 280 nm. FIGS. 9 show the results.
Note that, FIG. 9(a) shows a change in absorbance at OD 280 nm at the time of running pure water, and FIG. 9(b) shows a change in absorbance at OD 280 nm at the time of running the 30 mM ammonium acetate solution.

As shown in FIGS. 9, the results demonstrated that the recovery of the fractions in which the sialic acid-containing compound dissociated from the serotonin affinity support had been eluted was attained by running pure water through the column (a) to remove the component unadsorbed to the serotonin affinity support, stopping the running of the pure water for 12 hours, and then running the 30 mM ammonium acetate solution through the column (b). It was also shown that the sialic acid-containing compound was particularly abundantly eluted in the fractions at around 30 to 80 minutes after the start of running of the 30 mM ammonium acetate solution through the column.

### <Example 3> (Extraction from polished rice surface layer part with water and purification)

### [Serotonin affinity column purification]

15 mL of water were added to 3 g of flour derived from a part in the range of 91 to 86% by mass corresponding to a polished rice surface layer part when brown rice was regarded as 100% by mass (polished rice surface layer part recovered by grinding out), the part being obtained by grinding out brown rice (Koshihikari) successively from the outside using a grinding type rice milling machine (Grain Testing Mill manufactured by SATAKE), and the contents were mixed well.
The mixture was subjected to ultrasonication for 1 minute and 30 seconds. The treated product was left to stand still at 10°C for 12 hours and then centrifuged at 3000 rpm for 10 minutes to perform solid-liquid separation.
2 mL of the separated crude extract solution were applied to a column with a diameter of 26 mm and a length of 20 cm (XK26/20 manufactured by Pharmacia Bioteck) packed with the serotonin affinity support (LAS-Serotonin Support manufactured by J-Oil Mills), in which the support had been packed at a height of 4.6 cm, and was fractionated. The conditions for chromatography were as described below. Pure water was run at a flow rate of 0.2 mL/min for 300 minutes to remove the unadsorbed components (zone 1), and subsequently, the ammonium acetate solution with a linear gradient from 0 M to 1 M was run for 240 minutes to recover the sialic acid-containing compound dissociated from the support (zone 2). Note that, subsequently, the 1M ammonium acetate solution was run for 180 minutes to wash the column (removal of components adsorbed to the column) (zone 3), and finally the linear gradient solution from a 1 M ammonium acetate solution to pure water was run for 60 minutes, and then pure water was run for 180 minutes to regenerate the column (zone 4).
The sialic acid-containing compound was detected by measuring the amount of the compound eluted from the serotonin affinity support in terms of an absorbance at OD 280 nm. FIG. 10 shows the results.
FIG. 10 shows time-dependent changes in absorbance at OD 280 nm of the solutions which run through the serotonin affinity column.

Further, the fractionation with the serotonin affinity column was performed twice (twice using 2 mL of the crude extract solution each time). Fractions (zone 2) in which the ammonium acetate solution with a linear gradient from 0 M to 1 M had been run in twice of the fractionation were combined and freeze-dried with a freeze-dryer (Freezdryer FD-1 manufactured by EYELA), and the weights of the dried products were measured.

As shown in FIG. 10, the results demonstrated that the recovery of the fractions in which the sialic acid-containing compound dissociated from the serotonin affinity support had been eluted was attained by running the ammonium acetate solution with a linear gradient from 0 M to 1 M (zone 2: 300 to 540 minutes) through the column, after running pure water through the column to elute the components unadsorbed to the serotonin affinity support from the column (zone 1: 0 to 300 minutes). It was also shown that the sialic acid-containing compound dissociated from the serotonin affinity support was eluted in the fractions at around 660 minutes even in the fractions eluted by running the 1 M ammonium acetate solution for washing the column (zone 3: 540 to 720 minutes).
It was also shown that the sialic acid-containing compound was abundantly eluted particularly in the fractions at around 430 minutes (fractions at around 130 minutes after the start of the running of the ammonium acetate solution through the column in zone 2) as a peak in zone 2 and zone 3.
Note that, it is conceivable that the eluted peak of the sialic acid-containing compound was broad in zone 2 and zone 3 probably because the flow rate of the solution was slow relative to the volume of the column used in this example (column having a volume 40 times as large as those in Examples 1 and 2 was used) and thus the eluted peak was diffused.

Note that, from the fractions (zone 2) obtained by fractionating 4 mL (twice by 2 mL) of the crude extract solution using the serotonin affinity column, 0.5 to 0.9 mg of a dry solid was obtained, and thus, it was shown that 2.5 to 4.5 mg of a dry solid of the sialic acid-containing compound were obtained from 3 g of the flour of the polished rice surface layer part as the raw material (20 mL of the crude extract solution were obtained from 3 g of the flour of the polished rice surface layer part through the above-mentioned steps).

### [Resorcinol hydrochloric acid detection]

Next, the solutions which had run through the serotonin affinity column via the above-mentioned purification steps were fractionated and recovered as one fraction every 10 minutes to thereby recover 96 fractions in total, which were then freeze-dried.
Representative fractions in the above-mentioned zones 1 to 4 among those fractions were dissolved again in pure water, and the resultant solutions were developed with CH₃Cl/CH₃OH/0.2% CaCl₂=4/4/1 (V/V/V) and separated into individual components on a silica gel plate.
The silica gel plate on which the solutions had been developed was left to stand still under an iodine vapor atmosphere at room temperature for 72 hours to detect all organic compounds. Subsequently, the silica gel plate taken out of the iodine vapor atmosphere was left to stand still for 24 hours to desorb iodine sufficiently, sprayed with resorcinol hydrochloric acid, and heated at 95°C to develop a color, to thereby detect sialic acid-containing compounds. FIGS. 11 show the results. Note that, in FIGS. 11, FIG. 11 (a) shows the all organic compounds detected after the separation of the representative fractions in the zones 1 to 4 by the thin layer chromatography, and FIG. 11(b) shows the sialic acid-containing compounds detected after the separation of the representative fractions in the zones 1 to 4 by the thin layer chromatography.

Note that, in FIGS. 11, each lane shows a development on a slica gel plate, wherein lane 1 shows that of the fraction 12 (sample A) eluted at the time between 110 minutes and 120 minutes, lane 2 shows that of the fraction 44 (sample B) eluted at the time between 430 minutes and 440 minutes, lane 3 shows that of the fraction 48 (sample C) eluted at the time between 470 minutes and 480 minutes, lane 4 shows that of the fraction 54 (sample D) eluted at the time between 530 minutes and 540 minutes, lane 5 shows that of the fraction 80 (sample E) eluted at the time between 790 minutes and 800 minutes, and lane 6 shows that of the fraction 89 (sample F) eluted at the time between 880 minutes and 890 minutes, respectively.

As shown in FIG. 11(b), the results demonstrated that the sialic acid-containing compound was contained in each of the fraction 44 eluted at the time between 430 minutes and 440 minutes (sample B) (lane 2), the fraction 48 eluted at the time between 470 minutes and 480 minutes (sample C) (lane 3), and the fraction 54 eluted at the time between 530 minutes and 540 minutes (sample D) (lane 4).
It was also shown that the sialic acid-containing compound was not contained in the fraction 80 eluted at the time between 790 minutes and 800 minutes (sample E) (lane 5) or the fraction 89 eluted at the time between 880 minutes and 890 minutes (sample F) (lane 6).

Those results showed that the sialic acid-containing compound was contained in each of the fractions including the peak in zone 2 in which the ammonium acetate solution was run with a linear gradient from 0 M to 1 M through the column shown in FIG. 10 while the sialic acid-containing compound was not contained in a low peak observed in zone 4 which was a regeneration process of the column.
Note that, as shown by the results of comparison between FIG. 11 (a) and FIG. 11 (b), it was shown that the sialic acid-containing compound 'with extremely less contamination with contaminants (content of the contaminants was equal to or less than the detection limit of the all organic compounds detected under the iodine vapor atmosphere)' was recovered from fraction 44 (sample B) eluted at the time between 430 minutes and 440 minutes (lane 2), from fraction 48 (sample C) eluted at the time between 470 minutes and 480 minutes (lane 3), and from fraction 54 (sample D) eluted at the time between 530 minutes and 540 minutes (lane 4).
Further, the detected sialic acid-containing compounds migrated greatly from the origin. The fact suggested that the compounds were glycolipids as substances each having high affinity to a developing solvent.

### [Discussion]

Those results revealed that a fraction containing extremely less contaminants and containing the sialic acid-containing compound at a high concentration was obtained by extracting from the flour derived from the rice with only water without using any harmful organic solvent and purifying the extract with the serotonin affinity column.
Note that the sialic acid-containing compound was abundantly contained in the fraction 12 (sample A) (lane 1) eluted at the time between 110 minutes and 120 minutes which was the peak in the zone 1 in which the components unadsorbed to the serotonin affinity support had been removed from the column by running pure water through the column. This seems to be because the sialic acid-containing compound in an amount which was much more than an adsorbability of the serotonin affinity column used here for the sialic acid-containing compound was contained in the sample solution applied to the column.
Accordingly, if a larger scale of a column having a sufficient adsorbability for the sialic acid-containing compound, e.g., an industrial column having a volume of about 10 L or more for the serotonin affinity support is used, the sialic acid-containing compound is expected to be separated and recovered more efficiently.

### <Test Example 12> (Detection and identification of sialic acid-containing compound by lectin blot)

15 mL of ethylene glycol were added to 3 g of rice germ (Koshihikari), and the contents were mixed well.
The mixture was subjected to ultrasonication using an ultrasonic apparatus (B-42J Ultrasonic Cleaner manufactured by BRANSON) for 1 minute and 30 seconds. The treated product was left to stand still at 10°C for 12 hours and then centrifuged at 3000 rpm for 10 minutes to perform solid-liquid separation (sample 12).
The "centrifuged supernatant of the extract solution from the rice germ with ethylene glycol" (sample 12) obtained by the above-mentioned steps was developed with CH₃Cl/CH₃OH/0.2% CaCl₂=4/4/1 (V/V/V) or CH₃Cl/CH₃OH/water=4/4/1 (V/V/V) and separated into individual components on a silica gel plate. The silica gel plate was then dried with cold air.
The silica gel plate was immersed in a solution of poly (isobutyl methacrylate) in n-hexane/CH₃Cl=9/1 (V/V) (concentration: 2 g/500 mL) for 30 seconds, and dried with cold air.

Then, the silica gel plate treated with poly(isobutyl methacrylate) was immersed in a solution of TRITIC fluorescence-labeled MAA lectin (manufactured by E. Y. Labolatory) diluted to 100 times with a 0.01 M phosphate buffer (pH 7.2 to 7.4) containing 0.15 M sodium chloride and 1% BSA or a solution of TRITIC fluorescence-labeled SNA-Ilectin (manufactured by E.Y. Labolatory) diluted to 100 times with a 0.01 M phosphate buffer (pH 7.2 to 7.4) containing 0.15 M sodium chloride and 1% BSA in a plastic container, and shaken at room temperature for 48 hours to perform a lectin binding reaction.
Note that the 'MAA lectin' is a lectin which is specifically bound to an "N-acetylneuraminic acid α (2→3) Gal" structure, and the 'SNA-I lectin' is a lectin which is specifically bound to an "N-acetylneuraminic acid α (2→6) Gal" or "N-acetylneuraminic acid α (2→6) GalNAc" structure.
After the completion of the lectin binding reaction, the silica gel plate treated with poly(isobutyl methacrylate) was washed to remove the fluorescent lectin which was unbound to the objective compound. Subsequently, the sialic acid-containing compound was detected by observing a portion where fluorescence was observed, i.e., a site where the lectin specifically bound to the sialic acid was present. The results are shown by each combination of detection conditions in FIGS. 12A to 12D.

Note that FIG. 12A shows the results of a reaction of the TRITIC fluorescence-labeled MAA lectin with the silica gel plate developed with CH₃Cl/CH₃OH/0.2% CaCl₂=4/4/1 (V/V/V).
FIG. 12B shows the results of a reaction of the TRITIC fluorescence-labeled MAA lectin with the silica gel plate developed with CH₃Cl/CH₃OH/water=4/4/1 (V/V/V).
FIG. 12C shows the results of a reaction of the TRITIC fluorescence-labeled SNA-I lectin with the silica gel plate developed with CH₃Cl/CH₃OH/0.2% CaCl₂=4/4/1 (V/V/V).
FIG. 12D shows the results of a reaction of the TRITIC fluorescence-labeled SNA-I lectin with the silica gel plate developed with CH₃Cl/CH₃OH/water=4/4/1 (V/V/V).
In each of FIGS. 12A to 12D, the left-hand photograph is an image of the silica gel plates captured before the lectin binding reaction, and the right-hand photograph is an image of the silica gel plates captured after the lectin binding reaction.

As shown in FIGS. 12A to 12D, the results confirmed that portions which did not emit light before the fluorescent lectin binding reaction (left-hand photograph in each figure) emitted light after the fluorescent lectin binding reaction (right-hand photograph in each figure) (portions indicated by arrows). The light-emitting portion on the right-hand photograph in each figure indicates that the fluorescence-labeled MAA lectin or SNA-I lectin was specifically bound to the compound having the structure specific for N-acetylneuraminic acid as described above.
Specifically, FIGS. 12A and 12B (detection results by MAA lectin) showed that the compound having the "N-acetylneuraminic acid α (2→3) Gal" structure was present in each of the compounds developed on the silica gel plate and shown by the arrows.
In addition, FIGS. 12C and 12D (detection results by SNA-I lectin) showed that the compound having the "N-acetylneuraminic acid α (2→6) Gal" structure or "N-acetylneuraminic acid α (2→6) GalNAc" structure was present in each of the compounds developed on the silica gel plate and shown by the arrows.
Therefore, the results of this test example showed that the compound containing sialic acid (N-acetylneuraminic acid) was present in the solution extracted from the rice germ with ethylene glycol.
Further, the detected sialic acid-containing compounds migrated greatly from the origin. The fact suggested that the compounds were glycolipids as substances each having high affinity to a developing solvent.

### <Test Example 13> (Detection of sialic acid-containing compounds from crude extract solutions of cooked rice)

"Rice excluding a part corresponding to the range of 100 to 96% by mass (brown rice outermost layer part) when brown rice was regarded as 100% by mass and including the remaining germ and aleurone layer" (in other words, rice having a volume of 96 % by mass and including the remaining germ and aleurone layer, obtained by grinding out the brown rice outermost layer part), obtained by grinding out brown rice (Koshihikari) from the outside using a brush type rice milling machine (HRG-122 manufactured by Minoru Industry Co., Ltd.) was immersed in water at 20°C (samples 13-1 and 13-2) or 25°C (samples 13-3, 13-4) and left to stand still for 6 hours. The rice was lightly washed three times, 2 parts by mass of water were added to 2 parts by mass of the rice, and the mixture was cooked with a home rice cooker. 15 mL of water were added to 3 g of the cooked rice, and the contents were mixed well.
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
Equal amounts of ethanol were added to the separated crude extract solutions, and the contents were mixed well. After that, the mixtures were left to stand still at 10°C for 12 hours and then centrifuged at 3000 rpm for 10 minutes to perform solid-liquid separation.
The separated supernatants were developed with CH₃Cl/CH₃OH/water=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 13 shows the results.

Note that, in FIG. 13, 2 µL of a crude extract solution (sample 13-1) extracted in the above-mentioned steps using water as the extraction solvent from the cooked rice obtained by immersing the 'rice excluding the brown rice outermost layer and including the germ and the aleurone layer' in water at 20°C, leaving the rice to stand still for 6 hours, and then cooking the rice were developed in lane 1 on the silica gel plate. In lane 2, 4 µL of the same sample as that in lane 1 (sample 13-2) was developed on the silica gel plate.
In lane 3, 2 µL of a crude extract solution (sample 13-3) extracted in the above-mentioned steps using water as the extraction solvent from the cooked rice obtained by immersing the 'rice excluding the brown rice outermost layer and including the germ and the aleurone layer' in water at 25°C, leaving the rice to stand still, and then cooking the rice were developed on the silica gel plate. In lane 4, 4 µL of the same sample as that in lane 3 (sample 13-4) were developed on the silica gel plate.

As shown in FIG. 13, the results demonstrated that, even if the 'rice excluding the brown rice outermost layer and including the germ and the aleurone layer' was cooked, the crude extract solution containing the sialic acid-containing compound was obtained by extracting from the cooked rice with water.
Further, the detected sialic acid-containing compounds migrated greatly from the origin. The fact suggested that the compounds were glycolipids as substances each having high affinity to a developing solvent.

### <Test Example 14> (Detection of sialic acid-containing compounds from crude extract solutions using glycerol)

15 mL of 100% (v/v) glycerol were added to 3 g of flour obtained by milling wheat (Norin 61) whole grains or barley (Sachiho Golden, Betzes, Daishimochi, Ichibanboshi, Karasugane No. 1) whole grains with a mill (Ultracentrifugal Mill manufactured by MRK-RETSCH), and the contents were mixed well (samples 14-1 to 14-6).
Those mixtures were subjected to ultrasonication using an ultrasonic apparatus (B-42J Ultrasonic Cleaner manufactured by BRANSON) for 1 minute and 30 seconds. Those treated products were left to stand still at 10°C for 12 hours and then centrifuged at 3000 rpm for 10 minutes to perform solid-liquid separation.
The separated crude extract solutions were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 14 shows the results.

Note that, in FIG. 14, each lane shows a development on a slica gel plate, wherein lane 1 shows that of the crude extract solution obtained from the wheat Norin 61 whole grain flour using 100% (v/v) glycerol as the extraction solvent in the above-mentioned steps (sample 14-1), lane 2 shows that of the crude extract solution obtained from the barley Sachiho Golden whole grain flour using 100% (v/v) glycerol as the extraction solvent in the above-mentioned steps (sample 14-2), lane 3 shows that of the crude extract solution obtained from the barley Betzes whole grain flour using 100% (v/v) glycerol as the extraction solvent in the above-mentioned steps (sample 14-3), lane 4 shows that of the crude extract solution obtained from the barley Daishimochi whole grain flour using 100% (v/v) glycerol as the extraction solvent in the above-mentioned steps (sample 14-4), lane 5 shows that of the crude extract solution obtained from the barley Ichibanboshi whole grain flour using 100% (v/v) glycerol as the extraction solvent in the above-mentioned steps (sample 14-5), and lane 6 shows that of the crude extract solution obtained from the barley Karasugane No. 1 whole grain flour using 100% (v/v) glycerol as the extraction solvent in the above-mentioned steps (sample 14-6), respectively.

As shown in FIG. 14, the results demonstrated that the crude extract solution containing the sialic acid-containing compound was obtained by extracting from the wheat whole grain flour or the barley whole grain flour using 100% (v/v) glycerol as the extraction solvent.
Further, the detected sialic acid-containing compounds migrated greatly from the origin. The fact suggested that the compounds were glycolipids as substances each having high affinity to a developing solvent. Note that the sialic acid-containing compounds that did not migrate from the vicinity of the origin in the samples 14-1 to 14-6 (lanes 1 to 6) are estimated to be glycoproteins adsorbed onto the silica gel plate.

### <Test Example 15> (Detection of sialic acid-containing compounds from crude extract solutions of flours and processed products derived from various cereals)

15 mL of water were added to 3 g of flour obtained by milling wheat (Norin 61) whole grains or the barley (Sachiho Golden, Betzes, Daishimochi, Ichibanboshi, Karasugane No. 1) whole grains with a mill (Ultracentrifugal Mill manufactured by MRK-RETSCH), and the contents were mixed well (samples 15-1 to 15-6). In addition, 15 mL of water were added to 3 g of the wheat (Norin 61) short or barley (Sachiho Golden) short, and the contents were mixed well (samples 15-7 and 15-8). Also, 15 mL of water were added to 3 g of flour obtained by milling rice (Hatsushimo) germ with the mill (Ultracentrifugal Millmanufactured by MRK-RETSCH), and the contents were mixed well (sample 15-9).
Those mixtures were subjected to ultrasonication using an ultrasonic apparatus (B-42J Ultrasonic Cleaner manufactured by BRANSON) for 1 minute and 30 seconds. Those treated products were immediately centrifuged at 1000 rpm for 10 minutes to perform solid-liquid separation.
The separated crude extract solutions were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 15 shows the results.

Note that, in FIG. 15, each lane shows a development on a slica gel plate, wherein lane 1 shows that of the crude extract solution obtained from the wheat Norin 61 whole grain flour using water as the extraction solvent in the above-mentioned steps (sample 15-1), lane 2 shows that of the crude extract solution obtained from the barley Sachiho Golden whole grain flour using water as the extraction solvent in the above-mentioned steps (sample 15-2), lance 3 shows that of the crude extract solution obtained from the barley Betzes whole grain flour using water as the extraction solvent in the above-mentioned steps (sample 15-3), lane 4 shows that of the crude extract solution obtained from the barley Daishimochi whole grain flour using water as the extraction solvent in the above-mentioned steps (sample 15-4), lane 5 shows that of the crude extract solution obtained from the barley Ichibanboshi whole grain flour using water as the extraction solvent in the above-mentioned steps (sample 15-5), lane 6 shows that of the crude extract solution obtained from the barley Karasugane No. 1 whole grain flour using water as the extraction solvent in the above-mentioned steps (sample 15-6), lane 7 shows that of the crude extract solution obtained from the wheat Norin 61 short using water as the extraction solvent in the above-mentioned steps (sample 15-7), lane 8 shows that of the crude extract solution obtained from the barley Sachiho Golden short using water as the extraction solvent in the above-mentioned steps (sample 15-8), and lane 9 shows that of the crude extract solution obtained from the milled rice (Hatsushimo) germ flour using water as the extraction solvent in the above-mentioned steps (sample 15-9), respectively.

As shown in FIG. 15, the results demonstrated that the crude extract solution containing the sialic acid-containing compound was obtained by extracting from wheat or barley whole grain flour or short, or rice germ using water as the extraction solvent.
It was shown that one specific type of the sialic acid-containing compound was extracted from the rice germ at higher concentration compared with the wheat and barley whole grain flours and short. It was also shown that multiple types of the sialic acid-containing compounds which were not contained in the rice germ could be extracted from the wheat and the barley. Concerning the wheat and the barley, it was shown that a higher concentration of the sialic acid-containing compound was extracted using the short as the extraction raw material rather than using the whole grain flours.
Further, most of the detected sialic acid-containing compounds migrated greatly from the origin. The fact suggested that the compounds were glycolipids as substances each having high affinity to a developing solvent. Note that the sialic acid-containing compounds that did not migrate from the vicinity of the origin in the samples 15-1 to 15-9 (lanes 1 to 9) are estimated to be glycoproteins adsorbed onto the silica gel plate.

### <Test Example 16> (Influence of acid type on extraction)

15 mL of water, 11.83% (2 N) acetic acid, or 7.06% (2 N) hydrochloric acid were added to 3 g of wheat Norin 61 short, and the contents were mixed well. Those mixtures were subjected to ultrasonication using an ultrasonic apparatus (B-42J Ultrasonic Cleaner manufactured by BRANSON) for 1 minute and 30 seconds. Those treated products were shaken at 80°C for 3 hours (sample 16-1 to 16-3) and then centrifuged at 1000 rpm for 10 minutes to perform solid-liquid separation.
Also, 15 mL of water were added to 3 g of the wheat Norin 61 short, and the contents were mixed well. The mixture was subjected to ultrasonication using an ultrasonic apparatus (B-42J Ultrasonic Cleaner manufactured by BRANSON) for 1 minute and 30 seconds. The treated product was centrifuged at 1000 rpm for 10 minutes immediately (Sample 16-4) or after being left to stand still at 10 °C for 2.85 h (about 3 hours) (Sample 16-8), to perform solid-liquid separation.
The separated crude extract solutions were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby sialic acid-containing compounds. FIG. 16 shows the results.

Note that, in FIG. 16, each lane shows a development on a slica gel plate, wherein lane 1 shows that of the crude extract solution obtained by extracting from the wheat Norin 61 short using water as the extraction solvent, then ultrasonication followed by shaking at 80°C for 3 hours and subsequent centrifugation thereof (sample 16-1), lane 2 shows that of the crude extract solutionobtained by extracting from the wheat Norin 61 short using 2 N acetic acid as the extraction solvent, then ultrasonication followed by shaking at 80°C for 3 hours and subsequent centrifugation thereof (sample 16-2), and lane 3 shows that of the crude extract solution obtained by extracting from the wheat Norin 61 short using 2N hydrochloric acid as the extraction solvent, then ultrasonication followed by shaking at 80°C for 3 hours and subsequent centrifugation thereof (sample 16-3), respectively.
Also, the crude extract solution obtained by extracting from the wheat Norin 61 short using water as the extraction solvent and then ultrasonication immediately followed by centrifugation thereof (sample 16-4), and the crude extract solution obtained by extracting from the wheat Norin 61 short using water as the extraction solvent and then ultrasonication followed by being left to stand still at 10°C for about 3 hours and subsequent centrifugation thereof (sample 16-8) were developed in lanes 4 and 8, respectively on the silica gel plate.
In addition, a sialic acid-containing compound authentic GD1a (manufactured by SIGMA), a sialic acid-containing compound authentic GT1b (manufactured by SIGMA), and a sialic acid-containing compound authentic N-acetylneuraminic acid (manufactured by SIGMA) were developed in lanes 5, 6, and 7, respectively on the silica gel plate.

As shown in FIG. 16, the results demonstrated that the sialic acid-containing compound which was different from those extracted using water and migrated to higher positions on the silica gel plate was obtained by treatment with acetic acid or hydrochloric acid at 80°C for about 3 hours. Those results are thought to be because 'the molecular weight of the sialic acid-containing compound was reduced or the sialic acid-containing compound was fragmented' by the treatment with the acid plus heat to thereby reduce its polarity.
That is, it was suggested that the treatment with the acid plus heat was able to reduce the molecular weight or cause the fragmentation to enhance easily-soluble property (enhance the elution efficiency).
Further, most of the detected sialic acid-containing compounds migrated greatly from the origin. The fact suggested that the compounds were glycolipids as substances each having high affinity to a developing solvent. Note that the sialic acid-containing compounds that did not migrate from the vicinity of the origin in the samples 16-1, 16-2, 16-4, and 16-8 (lanes 1, 2, 4, and 8) are estimated to be glycoproteins adsorbed onto the silica gel plate.

### <Test Example 17> (Influence of extraction time on extraction at room temperature)

10 mL of water were added to 2 g of wheat (Shirasagi komugi) short, and the contents were mixed well. The mixture was subjected to ultrasonication using an ultrasonic apparatus (B-42J Ultrasonic Cleaner manufactured by BRANSON) for 1 minute and 30 seconds. The treated product was centrifuged at 1000 rpm for 10 minutes immediately (sample 17-1) or after shaking at 25°C for 1 hour (sample 17-2), 4 hours (sample 17-3), 12 hours (sample 17-4), or 24 hours (sample 17-5) to perform solid-liquid separation.
The separated crude extract solutions were developed with CH₃Cl/acetone/CH₃OH/acetic acid/water/pyridine=75/45/30/25/5/20 (V/V/V/V/V/V) and separated into individual components on a silica gel plate. The silica gel plate was sprayed with resorcinol hydrochloric acid and heated at 95°C to develop a color, to thereby detect sialic acid-containing compounds. FIG. 17 shows the results.

Note that, in FIG. 17, each lane shows a development on a slica gel plate, wherein lane 1 shows that of the crude extract solution obtained immediately after the ultrasonication after extracting from the wheat (Shirasagikomugi) short with water as the extraction solvent in the above-mentioned steps (sample 17-1), lane 2 shows that of the crude extract solution obtained after the ultrasonication followed by shaking at 25°C for 1 hour after extracting from the wheat (Shirasagikomugi) short with water as the extraction solvent in the above-mentioned steps (sample 17-2), lane 3 shows that of the crude extract solution obtained after the ultrasonication followed by shaking at 25°C for 4 hours after extracting from the wheat (Shirasagikomugi) short with water as the extraction solvent in the above-mentioned steps (sample 17-3), lane 4 shows that of the crude extract solution obtained after the ultrasonication followed by shaking at 25°C for 12 hours after extracting from the wheat (Shirasagikomugi) short with water as the extraction solvent in the above-mentioned steps (sample 17-4), and lane 5 shows that of the crude extract solution obtained after the ultrasonication followed by shaking at 25°C for 24 hours after extracting from the wheat (Shirasagikomugi) short with water as the extraction solvent in the above-mentioned steps (sample 17-5), respectively.

As shown in FIG. 17, the results demonstrated that the color-developed bands close to an origin were shifted to bands developed on upper positions by immersing the wheat short in water at 25°C (for 4 hours or more, particularly 12 hours or more). The result was obtained probably because 'the molecular weight of the sialic acid-containing compound was reduced or the sialic acid-containing compound was fragmented' to thereby reduce its polarity by an action of the endogenous enzyme.
That is, it was suggested that a reduction in molecular weight of the sialic acid-containing compound in the wheat short or the fragmentation of the sialic acid-containing compound (a change in composition of the sialic acid-containing compound) to enhance the easily-soluble property (enhance the elution efficiency) was attained without the addition of a specific enzyme.
Further, most of the detected sialic acid-containing compounds migrated greatly from the origin. The fact suggested that the compounds were glycolipids as substances each having high affinity to a developing solvent. Note that the sialic acid-containing compounds that did not migrate from the vicinity of the origin in the samples 17-1 to 17-5 (lanes 1 to 5) are estimated to be glycoproteins adsorbed onto the silica gel plate.

### <Test Example 18> (Detection and identification of sialic acid-containing compounds by lectin blot from crude extract solution of each cereal bran)

15 mL of water were added to 3 g of rice (Hitomebore) bran, wheat (Nishinochikara) bran, or barley (Mannenboshi) bran, and the contents were mixed well.
Those mixtures were subjected to ultrasonication using an ultrasonic apparatus (B-42J Ultrasonic Cleaner manufactured by BRANSON) for 1 minute and 30 seconds. Those treated products were immediately centrifuged at 1000 rpm for 10 minutes to perform solid-liquid separation (samples 18-1 to 18-3).
The crude extract solution obtained by the above-mentioned steps was developed with CH₃Cl/CH₃OH/0.2% CaCl₂=4/4/1 (V/V/V) and separated into individual components on a silica gel plate. The silica gel plate was then dried with cold air.
The silica gel plate was immersed in a solution of poly(isobutyl methacrylate) in n-hexane/CH₃Cl=9/1 (v/v) (concentration: 2 g/500 mL) for 30 seconds, and dried with cold air.

Then, the silica gel plate treated with poly(isobutyl methacrylate) was immersed in a solution of TRITIC fluorescence-labeled MAA lectin (manufactured by E. Y. Labolatory) diluted to 100 times with a 0.01 M phosphate buffer (pH 7.2 to 7.4) containing 0.15 M sodium chloride and 1% BSA, and shaken at room temperature for 24 hours to perform the lectin binding reaction.
Note that the 'MAA lectin' is a lectin which is specifically bound to an "N-acetylneuraminic acid α (2→3) Gal" structure.
After the completion of the lectin binding reaction, the silica gel plate treated with poly(isobutyl methacrylate) was washed to remove the fluorescent lectin which was unbound to the objective compound. Subsequently, the sialic acid-containing compound was detected by observing a portion where fluorescence was observed, i.e., a site where the lectin specifically bound to the sialic acid was present. FIG. 18 shows the results.

Note that FIG. 18 shows the results of a reaction of the TRITIC fluorescence-labeled MAA lectin with the silica gel plate developed with CH₃Cl/CH₃OH/0.2% CaCl₂=4/4/1 (V/V/V). That is, the left-hand photograph is an image of the silica gel plate captured before the lectin binding reaction, and the right-hand photograph is an image of the silica gel plate captured after the lectin binding reaction.
In FIG. 18, each lane shows a development on a slica gel plate, wherein lane 1 shows that of the crude extract solution obtained by extracting from the rice (Hitomebore) bran with water as the extraction solvent in the above-mentioned steps (sample 18-1), lane 2 shows that of the crude extract solution obtained by extracting from the wheat (Nishinochikara) bran with water as the extraction solvent in the above-mentioned steps (sample 18-2), and lane 3 shows that of the crude extract solution obtained by extracting from the barley (Mannenboshi) bran with water as the extraction solvent in the above-mentioned steps (sample 18-3), respectively.

As shown in FIG. 18, the results confirmed that in the portion in which light was not emitted before the fluorescent lectin binding reaction (left-hand photograph in the figure), light was emitted (portion indicated by arrows) after the fluorescent lectin binding operation (right-hand photograph in the figure). The light-emitting portion in the right-hand photograph in the figure indicates that the fluorescence-labeled MAA lectin was specifically bound to the compound having the structure specific for N-acetylneuraminic acid described above.
To be specific, the photograph indicates that the compound having the "N-acetylneuraminic acid α (2→3) Gal" structure is present.
Therefore, the results in this test example showed that the compound containing sialic acid (N-acetylneuraminic acid) was present in the extract solution of the rice bran, the wheat bran, or the barley bran extracted with water.
Further, the detected sialic acid-containing compounds migrated greatly from the origin. The fact suggested that the compounds were glycolipids as substances each having high affinity to a developing solvent.

### <Example 4> (Extraction from rice whole grains with methanol and gel filtration column purification)

### [Gel filtration column purification]

15 mL of methanol were added to 3 g of flour obtained by milling rice (Hitomebore) whole grains with a mill (Cyclone Sample Mill manufactured by UDY Corporation), and the contents were mixed well by hand with a glass rod. The treated product was immediately centrifuged at 3000 rpm for 10 minutes to perform solid-liquid separation. The supernatant obtained by the solid-liquid separation was concentrated with a nitrogen gas stream to afford a crude extract solution. The crude extract solution was fractionated with a high performance liquid chromatography system (Japan Analytical Industry, Co., Ltd., main body, differential refractive index detector) connected to a gel filtration column (Shodex). Methanol was used as an eluent.
As a result, as shown in FIG. 19, peaks 1 to 9 were obtained.

### [Resorcinol hydrochloric acid detection]

Next, the fractions represented by the above-mentioned peaks 1 to 9 were developed with CH₃Cl/CH₃OH/0.2% CaCl₂=4/4/1 (V/V/V) and separated into individual components on a silica gel plate. The silica gel plate was sprayed with resorcinol hydrochloric acid and heated at 95°C to develop a color, to thereby detect sialic acid-containing compounds. FIG. 20 shows the results. Further, lane numbers in FIG. 20 correspond to peak numbers in FIG. 19. Note that the crude extract solution before column fractionation was applied in lane S.
As shown in FIG. 20, the results demonstrated that the peak 8 contained the sialic acid-containing compound in a large amount.
Further, the detected sialic acid-containing compounds migrated greatly from the origin. The fact suggested that the compounds were glycolipids as substances each having high affinity to a developing solvent.

### [Discussion]

Those results showed that a fraction (purified product) containing the sialic acid-containing compound at a high concentration was obtained by extracting from rice with methanol and purifying the extract with a gel filtration column.

### Industrial Applicability

According to the present invention, there canbeprovided the sialic acid-containing compound which has no risk of contamination with pathogens affecting animals and has high safety from a plant-derived raw material, in particular, seeds of cereal or seeds of bean and a processed product of the seeds in an inexpensive manner.
Further, according to the present invention, there can be created novel applications for rice bran (red bran or white bran), wheat bran, soybean waste, irregular adzuki beans, and residue after extracting starch from corn, which are wastes derived from plants.
Further, the sialic acid-containing compound derived from the plant, which is the article of the present invention, can be utilized for functional foods, high value-added cosmetics, and pharmaceutical raw materials each containing sialic acid.

## Claims

1. An extraction method for a sialic acid-containing compound, the method comprising: crudely extracting a soluble component from a plant body or a processed product of the plant body with water, an alcohol, or an aqueous alcohol; and separating and recovering the sialic acid-containing compound from the resultant crude extract solution by dialysis, salt precipitation, or column chromatography.

2. An extraction method for a sialic acid-containing compound according to claim 1, whrerein the plant body comprises seeds of cereal and/or seeds of bean.

3. An extraction method for a sialic acid-containing compound according to claim 1 or 2, wherein the plant body or the processed product of the plant body has been milled, ground, triturated, or pulverized.

4. An extraction method for a sialic acid-containing compound according to any one of claims 1 to 3, wherein ultrasonication is performed in the step of crudely extracting the soluble component.

5. An extraction method for a sialic acid-containing compound according to any one of claims 1 to 4, wherein the water is tap water, deionized water, distilled water, or water containing a salt, an acid, an alkali, saccharides, or a pH buffer.

6. An extraction method for a sialic acid-containing compound according to any one of claims 1 to 5, wherein the alcohol is methanol, ethanol, isopropanol, ethylene glycol, or glycerol.

7. An extraction method for a sialic acid-containing compound according to any one of claims 1 to 6, wherein the separating and recovering are performed with a serotonin affinity column, a lectin affinity column, a gel filtration column, an ion-exchange column, or a silica gel column.

8. A sialic acid-containing compound, which is obtained by the extraction method according to any one of claims 1 to 7.
